# EUROPEAN PATENT APPLICATION

(11) **EP 1 712 566 A1**
(43) Date of publication of application: **18.10.2006**
(21) Application number: 05703804.4
(22) Date of filing: 19.01.2005
(51) Int. Cl.: C07K 16/28, C12N 15/09, C12Q 1/02, A61K 45/00, A61K 38/00, A61K 39/395, A61P 29/00, G01N 33/15, G01N 33/50

(54) **INFLAMMATORY CYTOKINE INHIBITOR**

(30) Priority: 19.01.2004 JP 2004010971
(71) Applicant: Medical and Biological Laboratories Co., Ltd., Nagoya-shi, Aichi 460-0002 (JP)
(72) Inventor: Okabe, Ayako, Kamiina-gun Nagano 3960215 (JP); Toji, Shingo, Ina-shi, Nagano 3960621 (JP); Kishi, Yoshiro, Ina-shi, Nagano 3994501 (JP); Yahara, Ichiro, Yokohoma-shi, Kanagawa 2220012 (JP)
(74) Representative: Denison, Christopher Marcus
(86) International application number: PCT/JP2005/000567
(87) International publication number: WO 2005/068504

(57) **Abstract**

A search was conducted for functional antibodies that endogenously regulate cytokines, focusing on the relationship between inflammatory diseases and cytokines. Mice were immunized with a human peripheral blood monocyte fraction and the obtained antibodies were examined for a cytokine-regulating effect. As a result, of these antibodies, antibody #33 was confirmed to inhibit the production of numerous typical inflammatory cytokines. When, at the same time, the promoting effect of antibody #33 on IL-10 production was examined, it was revealed that antibody #33 has an effect of accelerating IL-10 production but no activity to induce an excessive IL-10 production. The antigen of this promising antibody was verified to be CD61. Therefore, it is conceivable that when anti-CD61 antibodies are applied to the treatment of inflammatory diseases, they would become pharmaceuticals with both a definite efficacy and a high safety.

## Description

### Technical Field

The present invention relates to inflammatory cytokine inhibitors using an anti-CD61 antibody.

### Background Art

Inflammation is a complex series of reactions expressed by living organisms in response to stimuli. Causes of inflammation are varied: for example, exogenous factors including pathogenic microorganisms such as bacteria and viruses, physical factors such as external injury and radiation, and chemical factors such as acids and alkalis trigger inflammation. Inflammation is essentially one of the defense reactions of organisms. It can also be induced by endogenous causes: for example, immune complexes generated by endogenous autoantigens cause autoimmune diseases.

A large number of diseases accompany inflammatory symptoms, one example of which is sepsis. Sepsis is a disease showing a systemic inflammatory reaction in response to infection, often triggered by infection involving burns, external injury, highly invasive surgery, and diseases such as cancer and pneumonia. During sepsis, an abnormal increase in inflammatory cytokine concentration in blood is observed (hypercytokinemia). In serious cases, a self-destructive systemic inflammation occurs, thrombi are formed and multiple organs become dysfunctional in a short period of time. Death results in worst cases.

In spite of being such a serious disease, few effective treatment methods are available for sepsis. Fatal sepsis cases are numerous, and every year, 210,000 people die of sepsis in the United States. Sepsis is ranked as the number one cause of death in the intensive care unit apart from cardiac diseases. Only limited treatment methods are clinically applied at present, examples being endotoxin absorption-mediated blood purification methods, and administration of, as a pharmaceutical, activated protein C (Xigris^{™}, Eli Lilly and Company) having anticoagulant and anti-inflammatory effects.

Inflammatory cytokines are cytokines that promote inflammation. "Cytokine" is a general term for a variety of physically active substances responsible for intercellular signal transductions. Cytokines form a group of substances involved in regulating various biological functions by participating in intracellular signal transduction via specific receptors.

Examples of biological functions in which cytokines are involved include biological defense and immune responses (Non-Patent Document 1). Of these, inflammation which is primarily an organism's defense reaction is deeply associated with cytokines. Among cytokines, TNFα, IL-6, IL-1, IL-12, and the like act to promote inflammation, and therefore, are classified as inflammatory cytokines. In addition, cytokines having an activity to regulate the production of these inflammatory cytokines are referred to as anti-inflammatory cytokines (Non-Patent Documents 2 and 3). These inflammatory cytokines and anti-inflammatory cytokines are known to appropriately regulate their mutual production level and activities (Non-Patent Document 3).

IL-10 is an anti-inflammatory cytokine. Since IL-10 is expected to exert inhibitory effects on the production of the inflammatory cytokines TNFα and IFN-γ, clinical trials of IL-10 administration to psoriasis and Crohn's disease patients are being carried out, and some positive results have been obtained (Non-Patent Documents 8 and 9). However, in contrast to these findings, it has also been reported that administration of a large dose of exogenous IL-10 has the risk of aggravating inflammation (Non-Patent Document 10). Therefore, the treatment of inflammatory diseases aiming at the inhibitory effect of IL-10 on inflammatory cytokines is not easy.
[Non-Patent Document 1] Atsushi Miyajima, Toshio Kitamura and Naoko Arai, Molecular Biology of Cytokines, Yodosha 1995
[Non-Patent Document 2] Moore, K.W., et al., Annu. Rev. Immunol. 19:683-765, 2001
[Non-Patent Document 3] Mosmann, T.R. Adv. Immunol., 56:1-26, 1994
[Non-Patent Document 4] Shigeo Koyasu, Menekigaku ga wakaru (All about Immunology), pp74-80, Yodosha, 2000
[Non-Patent Document 5] Mosmann, T.R. Ann. NY Acad. Sci.,664:89-92, 1992
[Non-Patent Document 6] Bloom, B.R et al., Ann. Rev. Immunol., 10:453-488, 1997
[Non-Patent Document 7] Klingemann, H.G. and Dedhar, S., Blood 74(4):1348-1354, 1989
[Non-Patent Document 8] Kimball, A.B., et al., Arch Dermatol 138(10):1341-1346, 2002
[Non-Patent Document 9] Van Deventer, S.J.H., et al., Gastroenterology, 113: 383-389, 1997
[Non-Patent Document 10] Lauw, F.N., et al., The Journal of Immunology 2000, 165:2783-789

### Disclosure of the Invention

### Problems to be Solved by the Invention

The present invention was achieved in view of the above circumstances. An objective of the present invention is to provide a novel therapeutic drug for inflammatory diseases including sepsis.

### Means to Solve the Problems

To solve the above-described problems, the present inventors conducted a dedicated search for candidate substances of inflammatory disease therapeutic drugs. The present inventors, who focused on the relationship between inflammatory diseases and cytokines, came up with the idea of obtaining functional antibodies that would endogenously regulate cytokines. Mice were immunized with a human peripheral blood monocyte fraction (PBMC), and cytokine-regulating effects of the obtained antibodies were examined. As a result, it was confirmed that, of these antibodies, antibody #33 significantly inhibited IFN-γ production. At the same time, the present inventors confirmed the IL-10 production-promoting effect of antibody #33.

As described above, the present inventors further examined the IL-10 production-promoting effect of antibody #33 since results contrary to expectations are being reported for exogenous IL-10 administration. As a result, antibody #33 was found to have the effect of accelerating IL-10 production, but no activity to induce excessive IL-10 production.

Furthermore, when antibody #33 was further examined for regulatory effects on other cytokines, it was found to inhibit the production of many typical inflammatory cytokines.

These results showed that, in addition to having no risk of inducing inflammation due to excessive IL-10 production, antibody #33 of the present inventors definitely inhibits the production of typical inflammatory cytokines. If antibody #33 is applied to the treatment of inflammatory diseases, it may become a pharmaceutical having both a definite efficacy and a high safety. Furthermore, antigen investigations for this promising antibody revealed that the antigen is CD61. In other words, the present inventors discovered that a substance binding to CD61 possesses an inhibitory effect on inflammatory cytokine production and generated a novel therapeutic drug for inflammatory diseases using this substance. Specifically, the present invention provides the following:
(1) a substance, or a derivative thereof, having an ability to bind to a CD61 protein and an inhibitory effect on inflammatory cytokine production;
(2) the substance or derivative of (1), wherein the substance is a protein;
(3) the substance or derivative of (1), wherein the substance is an antibody;
(4) the substance or derivative of (1), (2), or (3), wherein the inflammatory cytokine is any one of IFN-γ, TNFα, IL-1, and IL-6;
(5) the substance or derivative of (1), (2), (3), or (4), having an IL-10 production-inducing effect;
(6) an inhibitor of inflammatory cytokine production comprising as an effective ingredient the substance or derivative of any one of (1) to (5);
(7) an isolated DNA of any one of the following (a) to (d):
   (a) a DNA of any one of SEQ ID NOs: 9 to 16;
   (b) a DNA encoding the amino acid sequence of any one of SEQ ID NOs: 1 to 8;
   (c) a DNA which hybridizes under stringent conditions with a DNA of any one of SEQ ID NOs: 9 to 16; and
   (d) a DNA encoding an amino acid sequence with a deletion, addition, insertion and/or substitution of one or more amino acids in the amino acid sequence of SEQ ID NOs: 1 to 8;
(8) a vector carrying the DNA of (7);
(9) a transformant carrying the DNA of (7) or the vector of (8);
(10) an anti-CD61 antibody, wherein the heavy chain polypeptide is a polypeptide of the following (a) or (b):
   (a) a polypeptide comprising the amino acid sequence of SEQ ID NO: 4; or
   (b) a polypeptide comprising an amino acid sequence with a deletion, addition, insertion and/or substitution of one or more amino acids in the amino acid sequence of SEQ ID NO: 4 and wherein the amino acid is functionally equivalent to the amino acid sequence of SEQ ID NO: 4;
(11) an anti-CD61 antibody comprising the amino acid sequence of the following (a) or (b) as an amino acid sequence of a CDR (complementarity determining region) of a heavy chain polypeptide:
   (a) an amino acid sequence of any one of SEQ ID NOs: 1 to 3; or
   (b) an amino acid sequence with a deletion, addition, insertion and/or substitution of one or more amino acids in the amino acid sequence of SEQ ID NOs: 1 to 3 and which is functionally equivalent as a CDR (complementarity determining region) to the amino acid sequence of any one of SEQ ID NOs: 1 to 3;
(12) an anti-CD61 antibody, wherein a light chain polypeptide is a polypeptide of the following (a) or (b):
   (a) a polypeptide comprising the amino acid sequence of SEQ ID NO: 8; or
   (b) a polypeptide comprising an amino acid sequence with a deletion, addition, insertion and/or substitution of one or more amino acids in the amino acid sequence of SEQ ID NO: 8 and wherein the amino acid is functionally equivalent to the amino acid sequence of SEQ ID NO: 8;
(13) an anti-CD61 antibody comprising the amino acid sequence of the following (a) or (b) as an amino acid sequence of a CDR (complementarity determining region) of a light chain polypeptide:
   (a) an amino acid sequence of any one of SEQ ID NOs: 5 to 7; or
   (b) an amino acid sequence with a deletion, addition, insertion and/or substitution of one or more amino acids in the amino acid sequence of SEQ ID NOs: 5 to 7 and which is functionally equivalent as a CDR (complementarity determining region) to the amino acid sequence of any one of SEQ ID NOs: 5 to 7;
(14) a pharmaceutical for preventing or treating an inflammatory disease, wherein the pharmaceutical comprises the inhibitor of inflammatory cytokine production of (6) or the anti-CD61 antibody of any one of (10) to (13);
(15) a pharmaceutical for preventing or treating hypercytokinemia, wherein the pharmaceutical comprises the inhibitor of inflammatory cytokine production of (6) or the anti-CD61 antibody of any one of (10) to (13);
(16) a method for inhibiting inflammatory cytokine production using the substance or derivative of any one of (1) to (5) or the anti-CD61 antibody of any one of (10) to (13);
(17) a method for judging the effectiveness of the pharmaceutical of (14) or (15) in treating an inflammatory disease or hypercytokinemia, wherein the method comprises the step of contacting a test sample with an anti-CD61 antibody;
(18) a kit for judging the effectiveness of the pharmaceutical of (14) or (15) in treating an inflammatory disease or hypercytokinemia; and
(19) a method of screening for a substance having an ability to bind to a CD61 protein and an inhibitory effect on inflammatory cytokine production, wherein the method comprises the steps of:
   (a) contacting an inducer of cytokine production and a test substance with a CD61-expressing cell; and
   (b) measuring the inflammatory cytokine level, comparing it with that of a control contacted with only the inducer of cytokine production, and selecting a test substance that reduced the cytokine level produced.

### Brief Description of the Drawings

Fig. 1 shows a time course of the inhibitory effect of antibody #33 on the production of inflammatory cytokines. The horizontal axis represents the time after addition of the antibody and the stimulant (pI:C) while the vertical axis represents the level of each cytokine.
Fig. 2 shows the inhibitory effect of antibody #33 on the production of inflammatory cytokines.
Fig. 3 shows the IL-10 production-inducing effect of antibody #33. The horizontal axis represents the time after addition of the antibody and the stimulant (pI:C) while the vertical axis represents the level of IL-10.
Fig. 4A shows the variation over time of the inhibitory effect of IL-10 on IFN-γ production, from the time of induction of cytokine production. The time from induction of cytokine production to IL-10 addition is shown.
Fig. 4B shows the involvement of IL-10 in the inhibition of IFN-γ production by antibody #33. Numbers in parentheses represent the concentration of antibody #33 or the concentration of IL-10-neutralizing antibody (ng/ml).
Fig. 5 shows the inhibitory effect of antibody #33 on the production of various inflammatory cytokines at 48 hours after induction of cytokine production, when the time from the induction of cytokine production until the addition of antibody #33 was changed. The time from induction of cytokine production to addition of antibody #33 is shown.
Fig. 6A shows the observation of human monocyte-like cells THP-1 with an optical microscope.
Fig. 6B shows the observation of THP-1 differentiated into activated macrophage-like cells with phorbol ester (PMA). Flattened cells are evidence of cell differentiation.
Fig. 6C shows a flow cytometry analysis using THP-1 and differentiated THP-1 (TPH-1 + PMA). The horizontal axis represents the cell number while the vertical axis represents FITC intensity.
Fig. 7A shows electrophoresis of the precipitate obtained when differentiated THP-1 cell surfaces were immunoprecipitated with antibody #33.
Fig. 7B shows flow cytometry analyses of PBMC using antibody #33 and anti-CD51 antibody or antibody #33 and anti-CD61 antibody.
Fig. 8 shows an evaluation of the effect of antibody #33 on platelet aggregation.
Fig. 9 shows the inhibitory effect of antibody #33 on IFN-γ production in the presence of platelets. Numbers in parentheses represent the PRP/PBMC ratio (volume ratio).
Fig. 10 shows the inhibitory effect of the F(ab')₂ fragment of antibody #33 on the IFN-γ production. Numbers in parentheses represent the antibody #33 concentration (µg/ml).
Fig. 11 shows the sequence of the heavy-chain variable region of antibody #33 and the position of the CDRs in this sequence. The bracketed regions are regions corresponding to CDR1 to 3.

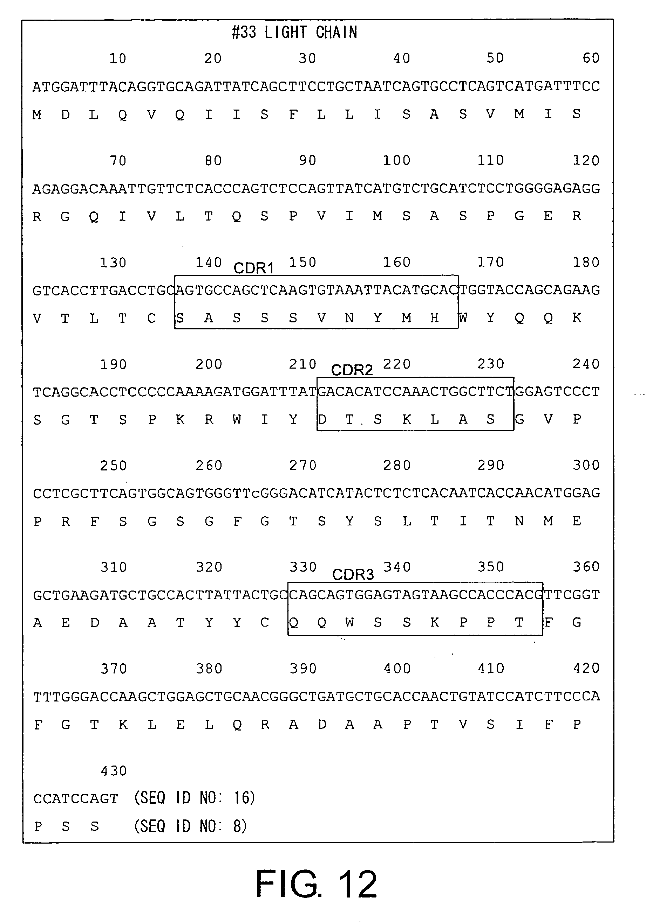

Fig. 12 shows the sequence of the light-chain variable region of antibody #33 and the position of the CDRs in this sequence. The bracketed regions are regions corresponding to CDR1 to 3.

### Best Mode for Carrying Out the Invention

The present invention provides substances (hereinafter referred to as "substances of the present invention"), or derivatives thereof, having a CD61 protein binding ability and an inhibitory effect on the production of inflammatory cytokines. The present invention is based on the discovery by the present inventors that an anti-CD61 antibody, or substances having a CD61 protein binding ability, inhibit the production of various inflammatory cytokines.

In general, "inflammatory cytokine" is a general term for cytokines causing inflammatory symptoms, good examples of which being IL-1, IL-6, IFN-γ, and TNF-α. In the present invention, the inhibitory effect on the production of inflammatory cytokines is not limited to the above-described examples and any one of the inflammatory cytokines can be inhibited. The substances may also have an inhibitory effect on the production of a plurality of inflammatory cytokines, either simultaneously or around the same time.

CD61 is a protein known as a cell surface molecule subunit and is also called integrin β₃-subunit and integrin β₃ chain. CD61 forms complexes with CD41 and CD51, i.e. CD41/CD61 and CD51/CD61, which function as cell surface molecules belonging to the integrin family. CD41/CD61 is also referred to as αIIbβ3, which is expressed specifically on platelets and megakaryocytes, functions in platelets as a receptor for fibrinogen and von Willebrand factors, and is a core factor in platelet aggregation and platelet adhesion. In addition, CD51/CD61 is also referred to as αVβ3 and is present on vascular endothelial cells, osteoclasts, macrophages, platelets, etc. These molecules are involved in cell adhesion, proliferation, and migration in the vascular endothelium and such, and also in angiogenesis, intimal hypertrophy of the blood vessels, etc. The binding region(s) and binding intensity do not matter when it comes to the CD61 protein binding ability according to the present invention, as long as an inflammatory cytokine inhibitory effect exists.

Since the substances of the present invention have an inhibitory effect on the production of inflammatory cytokines, administration of the substances may be effective in treating or preventing hypercytokinemia. Hypercytokinemia refers to a pathological condition in which cytokines in the blood have increased, and is observed in diseases such as sepsis, systemic inflammatory response syndrome (SIRS), hemophagocytosis syndrome (HPS), and Kawasaki disease, as well as in cases of surgical invasion. Furthermore, the substances of the present invention may be applied to the treatment or prevention of inflammatory diseases including sepsis. As described above, inflammatory cytokines are known to be involved in the establishment of inflammatory diseases, the aggravation of sepsis, etc. Inflammatory diseases refer to diseases showing inflammatory symptoms and are exemplified by sepsis and autoimmune diseases. Furthermore, systemic inflammatory response syndrome (SIRS), sepsis caused by infection, septic shock, and severe sepsis are all comprised in "sepsis" of the present invention.

Examples of such substances of the present invention include deoxyribonucleic acids, ribonucleic acids, proteins, peptides, and low-molecular substances. For example, fibrinogen which is a ligand for CD61, the RGD sequence which is its epitope, and derivatives thereof have a potential to become a substance of the present invention. As a preferred example, an anti-CD61 antibody may be given.

Antibodies are proteins also referred to as immunoglobulins. There are five classes of antibodies, IgM, IgD, IgG, IgE, and IgA. IgGs can be further divided into four subclasses: IgG1 to IgG4. Antibodies of every class have two heavy chains (H chains) and two light chains (L chains) as constituent units. The heavy chains and light chains are joined by disulfide bonds and noncovalent bonds, and these heavy chains are further joined by disulfide bonds and noncovalent bond(s) to constitute an antibody molecule. There are five types of H chains: µ, δ, γ, ε, and α which determine antibody class, while light chains are common to all classes and are either the κ chain or λ chain. Heavy chains consist of about 440 amino acid residues, while light chains consist of about one half of these amino acid residues. In both the heavy chains and light chains, the approximately 110 amino acid residue sequence from the N-terminal is highly variable between antibody molecules, and is thus called the "variable region". CDRs (complementarity determining regions), which determine antigenic specificity, exist in the variable region. There are three types of CDRs: CDR1, 2, and 3, each of which is a region of approximately five to ten amino acid residues. CDRs are located in the order of CDR1 to 3 from the N-terminal side, on both the heavy chains and light chains. CDRs are also referred to as "hypervariable regions". The variable region excluding the CDRs is referred to as the "frame work region", and is relatively highly conserved. The regions other than the variable regions are referred to as "constant regions", which are identical among antibodies of a same class or subclass.

The anti-CD61 antibodies of the present invention may be either polyclonal or monoclonal antibodies; however, they are preferably antibodies that are uniformly and stably produced. In addition, chimeric antibodies, humanized antibodies, human antibodies, antibody fragments, and mutants of the anti-CD61 antibodies are also examples of the anti-CD61 antibodies that can be used in the present invention. In the present description, a chimeric antibody is an antibody in which a human-derived constant region and a non-human animal-derived variable region are fused; and a humanized antibody is an antibody in which only a non-human animal-derived CDR(s) has been fused to a human-derived antibody lacking the CDR(s) (the constant regions and framework region(s)). Moreover, an antibody fragment refers to a portion of a full-length antibody and generally refers to a fragment comprising an antigen-binding region or a variable region. Fab, F(ab')₂, Fv, and scFv (single chain Fv) are preferable examples of antibody fragments of the present invention.

As long as the anti-CD61 antibodies of the present invention have an inhibitory effect on the production of inflammatory cytokines, it does not matter what epitope the antibody binds. Examples of such anti-CD61 antibodies include clone SZ21 and antibody #33. An example of a preferred antibody is antibody #33.

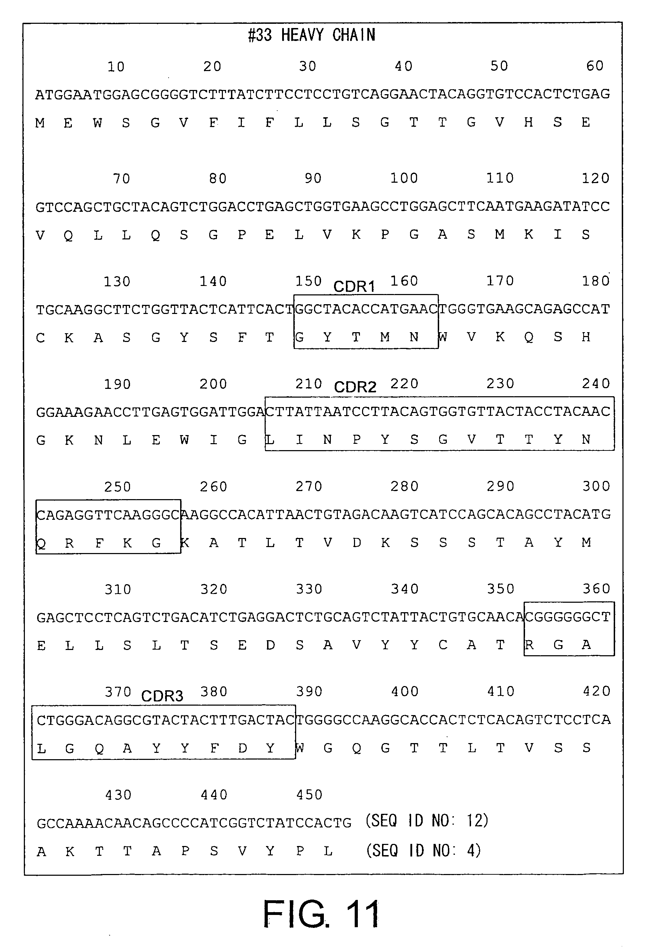

Antibody #33 of the present invention is a mouse anti-human CD61 antibody, for which the antigen is CD61. Of the anti-PBMC antibodies prepared by the present inventors, antibody #33's inhibitory effects on various inflammatory cytokines were specifically confirmed herein. Antibody #33 has been confirmed to work in an inhibitory manner towards at least IFN-γ, TNFα, IL-6, IL-1α, and IL-1β, as described in detail in the Examples. Furthermore, antibody #33 has an IL-10 production-inducing effect in the present invention as described below. The isotype of the antibody #33 is IgG2a, κ. When developed by SDS-PAGE (containing a reducing agent), bands can be observed at positions of approximately 55 kDa for the H chain and approximately 30 kDa for the L chain. Moreover, as mentioned later in the Examples, the amino acid sequences and cDNA sequences of the variable regions and CDR1 to 3 of antibody #33 were identified by the present inventors. These sequences are indicated below with the SEQ ID NOs. Furthermore, the positions of CDRs in the variable region is shown in Fig. 11.
SEQ ID NO: 1: antibody #33 heavy chain CDR1, amino acid sequence
SEQ ID NO: 2: antibody #33 heavy chain CDR2, amino acid sequence
SEQ ID NO: 3: antibody #33 heavy chain CDR3, amino acid sequence
SEQ ID NO: 4: antibody #33 heavy chain variable region, amino acid sequence
SEQ ID NO: 5: antibody #33 light chain CDR1, amino acid sequence
SEQ ID NO: 6: antibody #33 light chain CDR2, amino acid sequence
SEQ ID NO: 7: antibody #33 light chain CDR3, amino acid sequence
SEQ ID NO: 8: antibody #33 light chain variable region, amino acid sequence
SEQ ID NO: 9: antibody #33 heavy chain CDR1, DNA sequence
SEQ ID NO: 10: antibody #33 heavy chain CDR2, DNA sequence
SEQ ID NO: 11: antibody #33 heavy chain CDR3, DNA sequence
SEQ ID NO: 12: antibody #33 heavy chain variable region, DNA sequence
SEQ ID NO: 13: antibody #33 light chain CDR1, DNA sequence
SEQ ID NO: 14: antibody #33 light chain CDR2, DNA sequence
SEQ ID NO: 15: antibody #33 light chain CDR3, DNA sequence
SEQ ID NO: 16: antibody #33 light chain variable region, DNA sequence
Thus, antibody #33 can be described as "an anti-CD61 antibody, wherein the heavy chain polypeptide is a polypeptide comprising the amino acid sequence of SEQ ID NO: 4", "an anti-CD61 antibody comprising the amino acid sequences of SEQ ID NO: 1 to SEQ ID NO: 3 as the CDR (complementarity determining region) amino acid sequences of the heavy chain polypeptide", "an anti-CD61 antibody, wherein the light chain polypeptide is a polypeptide comprising the amino acid sequence of SEQ ID NO: 8", or "an anti-CD61 antibody comprising the amino acid sequences of SEQ ID NO: 5 to SEQ ID NO: 7 as the CDR (complementarity determining region) amino acid sequences of the light chain polypeptide".

Antibody #33 can be prepared according to known methods using human CD61 or human PBMC as antigen. In the case of polyclonal antibodies, mice can be injected with the antigen to produce the antibodies, and the collected blood can be purified by affinity chromatography. Monoclonal antibodies can be prepared by the hybridoma method (Kohler and Milstein, Nature 256:495 (1975)), the recombinant method (U.S. Patent No. 4816567) based on the sequence of a variable region elucidated by the present inventors, or such. Confirmation of whether the antibody thus prepared is antibody #33 can be performed by methods known to those skilled in the art. For example, an antibody can be identified as antibody #33 by determining the amino acid sequence of a variable region of the subject antibody using a protein sequencer and verifying whether it is identical to the amino acid sequence of SEQ ID NO: 4 or SEQ ID NO: 8. The DNA sequence of a variable region may also be determined using a DNA sequencer and compared with SEQ ID NO: 12 or SEQ ID NO: 16. Alternatively, the amino acid sequence or the DNA sequence of a CDR may be determined and compared with the sequences of SEQ ID NOs: 1 to 3, 5 to 7, 9 to 11, or 13 to 15.

Moreover, "an anti-CD61 antibody, wherein the heavy chain polypeptide is a polypeptide comprising the amino acid sequence of SEQ ID NO: 4" and "an anti-CD61 antibody, wherein the light chain polypeptide is a polypeptide comprising the amino acid sequence of SEQ ID NO: 8" comprise not only antibody #33, but also a modified antibody #33. Such a modified antibody #33 is also a preferable example of the anti-CD61 antibodies of the present invention. The aforementioned modified antibody #33 is characterized in that it comprises a variable region of antibody #33. Specifically, modified antibody #33 includes chimeric antibodies, or fragments thereof. Such a chimeric antibody is a chimeric antibody comprising a heavy chain variable region having the amino acid sequence of SEQ ID NO: 4, and a heavy chain constant region of a non-mouse animal, for example a human; or a chimeric antibody comprising a light chain variable region having the amino acid sequence of SEQ ID NO: 8, and a light chain constant region of a non-mouse animal, for example a human.

The above-described modified antibody #33 can be prepared by known methods. If the chimeric antibody is "an anti-CD61 antibody, wherein the heavy chain polypeptide is a polypeptide comprising the amino acid sequence of SEQ ID NO: 4" or "an anti-CD61 antibody, wherein the light chain polypeptide is a polypeptide comprising the amino acid sequence of SEQ ID NO: 8", the heavy chain variable region gene (SEQ ID NO: 4) of antibody #33 and the light chain variable region gene (SEQ ID NO: 8) of antibody #33 are prepared from antibody #33-producing hybridoma using known methods, or prepared by synthesis using a nucleic acid synthesizer. Antibodies of interest can be prepared by producing a vector by fusing this variable region gene with the constant region gene of an antibody derived from an animal such as a human and transfecting this expression vector into an appropriate host. The sequence of the constant portions of an antibody can be obtained from, for example, from Sequences of Proteins of Immunological Interest FIFTH EDITION 1991, Elvin A. Kabat et al, NIH Publication No. 91-3242. Descriptions of methods for preparing chimeric antibodies are mentioned in the Patent Document "Japanese Patent Application Kokai Publication No. (JP-A) H07-194384" and such. Furthermore, the various antibody fragments can also be prepared by known methods (for example, Osamu Kanemitsu, "Kotai Kogaku Nyumon (Handbook of Antibody Technology)", Nihon Gijutsushikai, ed., Chijinshokan, ISBN4-8052-0456-7).

Furthermore, "an anti-CD61 antibody comprising the amino acid sequences of SEQ ID NO: 1 to SEQ ID NO: 3 as the CDR (complementarity determining region) amino acid sequences of the heavy chain polypeptide" and "an anti-CD61 antibody comprising the amino acid sequences of SEQ ID NO: 5 to SEQ ID NO: 7 as the CDR (complementarity determining region) amino acid sequences of the light chain polypeptide" also comprise not only antibody #33, but also modified antibody #33. Such a modified antibody #33 is also a preferable example as an anti-CD61 antibody of the present invention. The above-described modified antibody #33 is characterized in that it comprises the CDRs of antibody #33. Specifically, it comprises a humanized antibody, or an antibody fragment thereof, comprising the amino acid sequences of SEQ ID NO: 1 to SEQ ID NO: 3 as the CDR (complementarity determining region) amino acid sequences of the heavy chain polypeptide and the amino acid sequences of SEQ ID NO: 5 to SEQ ID NO: 7 as the CDR (complementarity determining region) amino acid sequences of the light chain polypeptide.

The aforementioned humanized antibody can also be prepared by known methods. For example, an antibody of interest can be prepared by fusing the CDR gene (SEQ ID NO: 4) of the antibody #33 heavy chain and the CDR gene of the antibody #33 light chain, prepared by synthesis using a nucleic acid synthesizer, with genes of the framework and constant region of a human-derived antibody to generate an expression vector, and transfecting this expression vector into an appropriate host. The sequences of a human antibody constant portion and framework region can be obtained from, for example, "Sequences of Proteins of Immunological Interest FIFTH EDITION 1991, Elvin A. Kabat et al, NIH Publication No. 91-3242". Methods for preparing a humanized antibody are described in the Patent Documents "U.S. Patent No. 5225539," "European Patent No. 239400", "Japanese Patent No. 2912618", "Japanese Patent No. 2828340", "Japanese Patent Application Kokai Publication No. (JP-A) H11-4694", "Japanese Patent Application Kokai Publication No. (JP-A) H11-243955", the Non-Patent Document "Proc. Natl. Acad. Sci. USA 1989 vol. 86, p10029-10033, A humanized antibody that binds to the interleukin 2 receptor, Cary Queen et al.", etc. Furthermore, the various antibody fragments can also be prepared by known techniques.

Moreover, an anti-CD61 antibody mutant similar to antibody #33 is also a preferable example as an anti-CD61 antibody of the present invention. Examples of embodiments of such an antibody include "an anti-CD61 antibody, wherein the heavy chain polypeptide comprises an amino acid sequence with a deletion, addition, insertion and/or substitution of one or more amino acids in the amino acid sequence of SEQ ID NO: 4 and the amino acid sequence is functionally equivalent to the amino acid sequence of SEQ ID NO: 4" (hereinafter abbreviated as "heavy chain mutant") and "an anti-CD61 antibody, wherein the light chain polypeptide comprises an amino acid sequence with a deletion, addition, insertion and/or substitution of one or more amino acids in the amino acid sequence of SEQ ID NO: 8 and the amino acid sequence is functionally equivalent to the amino acid sequence of SEQ ID NO: 8" (hereinafter abbreviated as "light chain mutant"). Besides antibodies whose constant region is derived from mice, the above-described mutant comprises chimeric antibodies, humanized antibodies, human antibodies, and antibody fragments.

In "an amino acid sequence with a deletion, addition, insertion and/or substitution of amino acids" according to the present description, there is no particular limitation on the number of amino acids mutated, as long as the human CD61-recognizing function is maintained. In general, it is not more than 10% of the number of amino acids in the heavy chain or light chain variable region, preferably not more than 5%, and more preferably not more than 1%. There is no particular limitation on the amino acid mutation site in the heavy chain mutant and light chain mutant, as long as the human CD61-recognizing function is maintained, and may either be in the CDR or the framework region. It does not matter whether a light chain CDR mutation exists in a heavy chain mutant or a heavy chain CDR mutation exists in a light chain mutant. In other words, in the heavy chain mutant and the light chain mutant, the above-described mutations may exist in the heavy chain alone, in the light chain, or in both the heavy and light chains.

The above-described heavy chain mutants and light chain mutants can be prepared by known methods. For example, mutations can be introduced to the variable region DNA sequence of SEQ ID NO: 12 or SEQ ID NO: 16 using a known site-directed mutagenesis method such as deletion-mutagenesis or PCR method, or random mutagenesis. This variable region sequence can be ligated to a constant region gene derived from a mouse or from a non-mouse animal such as a human to construct an expression vector, and the above-described antibody of interest can be obtained using an appropriate host. Alternatively, known evolutionary molecular engineering techniques such as the chain shuffling method can be used. The chain shuffling method is a method of screening for combinations of variable regions of antibodies with a high specificity to the original antigen, in which the antibody variable region gene of either the heavy chain or the light chain is fixed, the other variable region gene is linked to a gene library of variable regions, and a library is constructed and expressed on phages. Whether the antibodies thus prepared are anti-CD61 antibodies can be confirmed by observing an antigen-antibody reaction using a known immunoassay and CD61 as antigen.

When the aforementioned anti-CD61 antibody mutant is a human antibody, it can be prepared by known techniques such as, methods using transgenic animals (Japanese Patent Application Kokai Publication No. (JP-A) H10-146194; Japanese Patent Application Kokai Publication No. (JP-A) H10-155492; Japanese Patent No. 2938569, Japanese Patent No. 3030092; Isao Ishida et al., Bioventure, Vol. 2, No. 4, 2002, p44-50) and phage display library method (Yuji Ito, Bioventure, Vol. 2, No. 4, 2002, p51-58; Toshihiro Nakajima, Bioventure, Vol. 2, No. 4, 2002, p59-66).

Furthermore, examples of other embodiments of an anti-CD61 antibody mutant similar to antibody #33 comprise "an anti-CD61 antibody comprising the amino acid sequence of (a) or (b) below as the CDR (complementarity determining region) amino acid sequence of the heavy chain polypeptide:
(a) an amino acid sequence of any one of SEQ ID NO: 1 to SEQ ID NO: 3; and
(b) an amino acid sequence with a deletion, addition, insertion, and/or substitution of one or more amino acids in the amino acid sequence of any one of SEQ ID NO: 1 to SEQ ID NO: 3 and which functions as a CDR (complementarity determining region) in the same way as the amino acid sequence of any one of SEQ ID NO: 1 to SEQ ID NO: 3" (hereinafter abbreviated as "heavy chain CDR mutant"). This mutant is characterized in that the amino acid sequences of CDR1 to 3 of the heavy chain are amino acid sequences selected from sequences of CDR1 to 3 of antibody #33 heavy chain and the amino acid sequences of CDR1 to 3 of antibody #33 heavy chain into which mutations have been added. Furthermore, examples of other embodiments comprise "an anti-CD61 antibody comprising the amino acid sequence of (a) or (b) below as the CDR (complementarity determining region) amino acid sequence of the light chain polypeptide:
   (a) an amino acid sequence of any one of SEQ ID NO: 5 to SEQ ID NO: 7; and
   (b) an amino acid sequence with a deletion, addition, insertion, and/or substitution of one or more amino acids in the amino acid sequence of any one of SEQ ID NO: 5 to SEQ ID NO: 7 and which functions as a CDR (complementarity determining region) in the same way as the amino acid sequence of any one of SEQ ID NO: 5 to SEQ ID NO: 7" (hereinafter abbreviated as "light chain CDR mutant"). This mutant is characterized in that the amino acid sequences of CDR1 to 3 of the light chain are amino acid sequences selected from sequences of CDR1 to 3 of antibody #33 light chain and the amino acid sequences of CDR1 to 3 of antibody #33 light chain into which mutations have been added.

There is no limitation on the CDR mutation sites in the heavy chain CDR mutants and the light chain CDR mutants, as long as the human CD61-recognizing function is maintained. Furthermore, the mutation of the CDR may exist on only one of CDR1 to 3, on any two, or on all three of them. It does not matter whether a light chain mutation exists in a heavy chain CDR mutant or whether a heavy chain mutation exists in a light chain CDR mutant.

Preparation of the heavy chain CDR mutants and the light chain CDR mutants as well as confirmation that they are anti-CD61 antibodies can be carried out by known methods similar to the above-described methods for preparing the heavy chain mutants and the light chain mutants.

The anti-CD61 antibodies can be prepared by known methods using CD61 or PBMC as antigen. In the case of polyclonal antibodies, antibodies can be produced by injecting animals such as rabbits and rats with the antigen and the collected blood can be purified by affinity chromatography. In the case of monoclonal antibodies, the hybridoma method (Kohler and Milstein, Nature 256: 495(1975)), the recombinant method (U.S. Patent No. 4816567), or such may be used. Moreover, anti-CD61 antibody mutants can be prepared by random mutagenesis, in which random mutations are introduced into specific genetic DNAs by the PCR method and such to produce mutants, or by the chain shuffling method, which screens for combinations of variable regions of antibodies with a high specificity to the original antigen, and in which the antibody variable region of either the VH gene or the VL gene is fixed and the other is linked to a V gene library to construct and express a library. F(ab')₂ are prepared by digesting IgG with pepsin and removing the Fc portions. In this case, the pH, the duration of digestion, and the amount of pepsin are appropriately adjusted.

Whether the prepared antibody is an anti-CD61 antibody can be confirmed by examining the affinity with the CD61 antigen. Confirmation of the affinity with the antigen is usually performed as a screening of the antibody at the time of preparation of the antibody. The affinity of an antibody can be determined by saturation binding, enzyme-linked immunosorbent assay (ELISA), or such.

Other substances of the present invention can be prepared by known methods in addition to the screening method described below. For example, when the substance of the present invention is a protein, proteins that bind to the CD61 protein can be screened from a group of candidate substances by known methods such as the West-western method, the two-hybrid method, the immunoprecipitation method, or such. Screening can also be performed using an apparatus (for example BIACORE) for analyzing the intermolecular interactions by surface plasmon resonance (SPR). Substances screened in this way can be further selected after confirming their inhibitory effect on the production of inflammatory cytokines and purified using affinity columns and such to prepare a substance of the present invention. The verification of the inhibitory effect on the production of inflammatory cytokines can be done as described in the Examples, by contacting a subject substance with inflammatory cytokine-producing cells, comparing the production level of the inflammatory cytokines by these cells before and after the contact with the subject substance, and examining whether the production level after the contact has decreased compared to before the contact. The level of inflammatory cytokines can be measured by various immunoassays that use anti-cytokine antibodies, such as the HPLC method and the ELISA method.

Furthermore, when the substance of the present invention to be prepared is a small molecule, it can be prepared, for example, by subjecting the small molecules of a library constructed by combinatorial chemistry to a high throughput screening by known methods (Wrighton NC; Farrell FX; Chang R; Kashyap AK; Barbone FP; Mulcahy LS; Johnson DL; Barrett RW; Jolliffe LK; Dower WJ.; Small peptides as potent mimetics of the protein hormone erythropoietin, Science (UNITED STATES) Jul 26 1996, 273 p458-64; Verdine GL., The combinatorial chemistry of nature, Nature (ENGLAND) Nov 7 1996, 384 p11-13; Hogan JC Jr., Directed combinatorial chemistry, Nature (ENGLAND) Nov 7 1996,384 p17-9).

The substances of the present invention may also have an IL-10 production-inducing effect. The IL-10 production-inducing effect of the present invention comprises the effect of accelerating the initiation time point of IL-10 production and secretion from IL-10 producing cells. When the effect of accelerating the initiation time point of IL-10 production and secretion is confirmed, the substance is judged to have the IL-10 production-inducing effect of the present invention even if the total amount of IL-10 production at the time of addition or administration of the substance did not increase compared to when no substance was added or administered. Confirmation of the IL-10 production-inducing effect can be done, as described in the Examples, by measuring the amount of IL-10 secreted from IL-10 producing cells by various known immunoassays such as the ELISA method using an anti-IL-10 antibody. Furthermore, the presence of the effect of accelerating the initiation time point of IL-10 production and secretion can be verified by monitoring over a period of time.

The present invention also provides derivatives of the above-described substances of the present invention. Examples of derivatives of the substances of the present invention include prodrugs of the substances of the present invention. Such prodrugs are substances for which the derivative itself has no CD61 protein-binding ability and/or no inflammatory cytokine production-inhibitory effect, but which become substances exhibiting a CD61 protein-binding ability and an inflammatory cytokine production-inhibitory effect after administration into the body. The prodrug derivatives of the above-described substances are considered to be useful in treating or preventing inflammatory diseases and hypercytokinemia, similarly to the above-described substances.

When the substances of the present invention, or derivatives thereof, are used as pharmaceuticals, they can be appropriately formulated according to standard methods. These formulations may comprise pharmacologically acceptable carriers and additives. For example, surfactants, excipients, stabilizers, preservatives, suspending agents, isotonizing agents may be comprised. Considering the administration route and the patient, a suitable dosage form can be selected among known dosage forms such as injections, tablets, capsules, microcapsules, etc.

The present invention provides DNAs which encode the variable regions of antibody #33. As described above, cDNAs of variable regions of antibody #33 have been identified by the present inventors. The heavy chain variable region and the light chain variable region of antibody #33 are both regions involved in determining the specificity of antibody #33 as an anti-CD61 antibody. As mentioned above, the amino acid sequence and cDNA sequence of a variable region of the antibody #33 heavy chain are described in SEQ ID NO: 4 and SEQ ID NO: 12 respectively, while the amino acid sequence and cDNA sequence of a variable region of the antibody #33 light chain are described in SEQ ID NO: 8 and SEQ ID NO: 16 respectively.

The above-described cDNAs of the variable regions of antibody #33 can be prepared by methods known to those skilled in the art. mRNAs can be prepared from antibody #33-producing hybridomas by known methods and DNAs of interest can be prepared from these mRNAs by the RT-PCR method. Specifically, they can be prepared according to the method described in the Examples. They can also be prepared by synthesis using a DNA synthesizer, based on the sequence of SEQ ID NO: 12 or SEQ ID NO: 16.

The present invention provides DNAs similar to cDNAs of the antibody #33 variable regions. Such DNAs comprise DNAs that hybridize under stringent conditions with the DNA of SEQ ID NO: 12 or SEQ ID NO: 16. Furthermore, they comprise DNAs which encode amino acid sequences with a deletion, addition, insertion, and/or substitution of one or more amino acids in the amino acid sequence of SEQ ID NO: 4 or SEQ ID NO: 8. Such DNAs comprise cDNAs of the variable region of mouse anti-CD61 antibodies other than antibody #33, cDNAs of the variable region anti-CD61 antibodies derived from non-mouse animals including human anti-CD61 antibodies, and artificially-produced variable region cDNAs of antibody #33 mutants.

DNAs similar to the cDNAs encoding the variable regions of the above-described antibody #33 can be prepared by known methods. For example, they can be prepared by isolation from cDNAs of various anti-CD61 antibodies other than antibody #33 by the hybridization technique, and using as a probe, a sequence of any one of SEQ ID NOS: 9 to 16, or a portion thereof. Furthermore, they can be prepared from anti-CD61 antibody mRNAs by the PCR method using a portion of the sequence of SEQ ID NO: 9 or 16 as a primer. Furthermore, they can be prepared by applying known gene modification methods to the sequence of SEQ ID NO: 12 or 16. Preparation using a DNA synthesizer is also possible.

Those skilled in the art can appropriately select the above-mentioned stringent hybridization conditions. For example, pre-hybridization is carried out in a hybridization solution containing 25% formamide (50% formamide under more stringent conditions), 4x SSC, 50 mM Hepes (pH 7.0), 10x Denhardt's solution, and 20 µg/ml denatured salmon sperm DNA at 42°C overnight. A labeled probe is added and hybridization is carried out by incubation at 42°C overnight. Post-hybridization washes are carried out under different levels of stringency including moderately stringent "1x SSC, 0.1% SDS, 37°C", highly stringent "0.5x SSC, 0.1% SDS, 42°C", and more highly stringent "0.2x SSC, 0.1% SDS, 65°C" conditions. As the stringency level of post-hybridization washes increases, DNA more highly homologous to the probe sequence is expected to be isolated. The above-described combinations of SSC, SDS, and temperature are merely examples of wash conditions. Those skilled in the art can achieve the same stringencies as those described above by appropriately combining the above factors or others (such as probe concentration, probe length, hybridization period, etc) that affect hybridization stringency.

A polypeptide thus isolated using hybridization will usually comprise an amino acid sequence highly homologous to the polypeptides identified by the present inventors. "Highly homologous" refers to sequence homology of at least 40% or more, preferably 60% or more, further preferably 80% or more, further preferably 90% or more, further preferably at least 95% or more, further preferably at least 97% or more (for example, 98% to 99%). Amino acid sequence identity can be determined, for example, using the BLAST algorithm according to Karlin and Altschul (Proc. Natl. Acad. Sci. USA. 87:2264-2268, 1990; Proc. Natl. Acad. Sci. USA. 90: 5873-5877, 1993). A program designated BLASTX has been developed based on this algorithm (Altschul et al., J. Mol. Biol. 215: 403-410, 1990). For analysis of amino acid sequence identity, the BLASTX program uses, for example, a score of 50 and a word length of 3 as parameters. When using BLAST and Gapped BLAST programs, the default parameters of the respective program are used. Specific methodology for performing BLAST analyses is publicly available at http://www.ncbi.nlm.nih.gov.

The above-described cDNAs of the antibody #33 variable regions and DNAs similar to the cDNAs of the antibody #33 variable regions can be used to produce antibody #33, antibody #33 heavy chain mutants, antibody #33 light chain mutants, chimeric antibodies thereof, antibody fragments thereof, and anti-CD61 antibody mutants similar to antibody #33. For the above-described production, the aforementioned cDNAs of antibody #33 variable regions or DNAs resembling the cDNAs of antibody #33 variable regions may be inserted into a vector and then introduced into host cells, and the antibody of interest can be obtained from the transformants into which the above-described cDNAs or vectors have been introduced. The vectors can be selected according to the purpose or host cell, and the constant region DNA may be inserted therein in advance. Specific examples of the vector comprise the plasmid pVk1. They can be used as probes to detect anti-CD61 antibodies.

The present invention provides DNAs encoding the CDRs of antibody #33. As described above, the cDNAs for antibody #33 CDRs were identified by the present inventors. Both the heavy chain CDR1 to 3 and the light chain CDR1 to 3 of antibody #33 are the most important regions involved in determining the specificity of antibody #33 as an anti-CD61 antibody. As mentioned above, the amino acid sequence and cDNA sequence of the variable CDR1 of antibody #33 heavy chain are described in SEQ ID NO: 1 and SEQ ID NO: 9 respectively; the amino acid sequence and cDNA sequence of the variable CDR2 of antibody #33 heavy chain are described in SEQ ID NO: 2 and SEQ ID NO: 10 respectively; the amino acid sequence and cDNA sequence of the variable CDR3 of antibody #33 heavy chain are described in SEQ ID NO: 3 and SEQ ID NO: 11 respectively; the amino acid sequence and cDNA sequence of the variable CDR1 of antibody #33 light chain are described in SEQ ID NO: 5 and SEQ ID NO: 13 respectively; the amino acid sequence and cDNA sequence of the variable CDR2 of antibody #33 light chain are described in SEQ ID NO: 6 and SEQ ID NO: 14 respectively; the amino acid sequence and cDNA sequence of the variable CDR3 of antibody #33 light chain are described in SEQ ID NO: 7 and SEQ ID NO: 15 respectively.

cDNAs of the above-described antibody #33 CDRs can be prepared by methods known to those skilled in the art. For example, they can be prepared by synthesis using a DNA synthesizer based on a sequence of any one of SEQ ID NO: 9 to SEQ ID NO: 11 or any one of SEQ ID NO: 13 to SEQ ID NO: 15.

The present invention provides DNAs similar to the cDNAs of the CDRs of antibody #33 variable regions. Such DNAs comprise DNAs that hybridize under stringent conditions with a DNA of any one of SEQ ID NO: 9 to SEQ ID NO: 11 and SEQ ID NO: 13 to SEQ ID NO: 15. Furthermore, they comprise DNAs which encode amino acid sequences with a deletion, addition, insertion, and/or substitution of one or more amino acids in the amino acid sequence of any one of SEQ ID NO: 1 to SEQ ID NO: 3 and SEQ ID NO: 5 to SEQ ID NO: 7. Such DNAs comprise cDNAs of the CDRs of mouse anti-CD61 antibodies other than antibody #33, cDNAs of the CDRs of anti-CD61 antibodies derived from animals other than mice and comprising human anti-CD61 antibodies, and cDNAs of the CDRs of artificially-produced antibody #33 mutants.

DNAs similar to the cDNAs of the CDRs of the above-described antibody #33 can be prepared by methods known to those skilled in the art. They can be prepared by applying the above-described known gene modification methods to a sequence of any one of SEQ ID NO: 9 to SEQ ID NO: 11 and SEQ ID NO: 13 to SEQ ID NO: 15. They can also be prepared using a DNA synthesizer. They can also be prepared by isolation from cDNAs of various anti-CD61 antibodies other than antibody #33 using a DNA of any one of SEQ ID NO: 9 to SEQ ID NO: 11 or SEQ ID NO: 13 to SEQ ID NO: 15 as a probe.

The above-described cDNAs of the CDRs of antibody #33 and DNAs similar to the cDNAs of the CDRs of antibody #33 can be used to produce antibody #33, the heavy chain CDR mutants of antibody #33, the light chain CDR mutants of antibody #33, humanized antibodies thereof, fragments of these antibodies which are human antibodies, and anti-CD61 antibody mutants similar to antibody #33. Furthermore, they can also be used to generate transgenic animals for human antibody production. For the above-described production, the aforementioned cDNAs of the CDRs of antibody #33 or DNAs similar to the cDNAs of the CDRs of antibody #33 may be inserted into a vector and then introduced into host cells, and the antibody of interest can be obtained from the transformants into which the above-described cDNAs or vectors have been introduced. The vectors can be selected according to the purpose or host cells and the DNA of the constant region or framework may be inserted therein in advance. Specific examples of the vector comprise the plasmid pVk1. Moreover, they can be used as probes to detect anti-CD61 antibodies.

The present invention provides methods for inhibiting the production of inflammatory cytokines using a substance of the present invention or a derivative thereof. The methods of the present invention can be performed *in vivo* or *in vitro.* For example, a substance of the present invention or a derivative thereof can be administered to experimental animals or cultured cells to inhibit the production of inflammatory cytokines in these experimental animals or cultured cells. The methods of the present invention may thereby become methods for elucidating biological reactions in which cytokines are involved. Furthermore, as described above, these methods may become effective methods, not only in the field of research, but also in the field of medicine. In other words, by administering a substance of the present invention, or a derivative thereof, to patients with inflammation or hypercytokinemia or patients with such predispositions, the substance or derivative can be used to prevent and treat inflammatory diseases and hypercytokinemia.

Moreover, the present invention provides a method for judging the efficacy of the above-described pharmaceuticals in treating inflammatory diseases or hypercytokinemia. The method of judgment of the present invention enables one to judge whether the treatment with the above-described pharmaceutical is effective or not for each patient prior to *in vivo* treatment, and can provide the clinical scene with so called tailor-made treatment. Thrombasthenia can be given as an example of a disorder whose association with a CD61 mutation has been clarified. Due to a type of CD61 mutation affecting fibrinogen-binding, platelet aggregation is inhibited, thus causing a tendency to bleed. The above-described CD61 mutation is known to have a single amino acid sequence mutation. Types D 119Y (amino acid 119 is mutated from D to Y; same is true for the other types), R214Q, R214W, and S752P have so far been identified. In addition, L33P, R143Q, P407A, R439Q, and R636C are known as asymptomatic mutations. If disease onset is related to inflammatory cytokines, it is conceivable that the methods of the present invention can likely be applied.

The methods of the present invention comprise a step of contacting a test sample with an anti-CD61 antibody.

An example of a method of the present invention is as follows: first, an inducer of cytokine production is added to peripheral blood or peripheral blood monocytes collected from a subject (hereinafter referred to as subject's sample) to induce inflammatory cytokine production. As an inducer of cytokine production, for example, LPS can be used. Next, the subject's sample treated as described above is divided, and an anti-CD61 antibody is added to one part while the other is used as a control. The cytokine production level in both samples is measured by ELISA or such. The cytokine to be measured can be selected from inflammatory cytokines. When the inflammatory cytokine level in the sample to which the anti-CD61 antibody has been added is lower than that in the control, the treatment with the above-described pharmaceutical is judged to have been effective.

As a different example, the level of cytokine production in a subject's sample is measured, then an anti-CD61 antibody is added to this sample, and then the level of cytokine production in the sample after antibody addition is measured by ELISA, or such. The measured values before and after antibody addition are compared, and when the value after antibody addition is lower than that before antibody addition, the treatment with the above-described pharmaceutical is judged to have been effective. This method can be applied to, for example, patients in whom an increase of the level of cytokines in blood has already been confirmed.

Although peripheral blood or peripheral blood monocytes has been cited as a preferred example of a subject's sample, the test sample is not limited thereto, and affected tissues in which an inflammation is occurring and such may be used.

Reagents necessary for a method of the present invention may be combined in advance as a kit. One of the reagents constituting the kit comprises an anti-CD61 antibody as an ingredient. Besides this, the kit may comprise a reagent for measuring a cytokine, a cytokine inducer, an appropriate diluent, etc, as required. Although they may vary according to the method for measuring cytokines, for example in the case of the ELISA method, the reagents for measuring cytokines are exemplified by reagents comprising as an ingredient an anti-cytokine antibody, an enzyme-labeled antibody, or a substrate.

The present invention provides methods of screening for the above-described substances of the present invention. A screening method of the present invention comprises the steps of: contacting CD61-expressing cells with an inducer of cytokine production and a test substance; and measuring the inflammatory cytokine level, comparing with a control contacted only with the inducer of cytokine production, and selecting a test substance that reduced the produced cytokine level.

"The step of contacting CD61-expressing cells with an inducer of cytokine production and a test substance" is explained: For example, CD61-expressing cells are seeded into a 96-well plate and an inducer of cytokine production and a test substance are added to each well simultaneously or in tandem to contact these substances with CD61-expressing cells. Herein, a test substance is a substance that is the object of the screening. Examples of CD61-expressing cells include PBMC, and examples of an inducing substance of cytokine production include LPS. In addition, CD61-expressing cells contacted with only the substance inducing cytokine production, without contacting the test substance, are prepared as a control. Next, the "step of measuring the inflammatory cytokine level, comparing with a control contacted only with the inducer of cytokine production, and selecting a test substance that reduced the produced cytokine level" is explained. When referring to the above-described example, the level of inflammatory cytokine in the supernatant of each well is measured by ELISA or such. The cell supernatant of the control is similarly measured. To "contact only with the inducer of cytokine production" means that the inducer of cytokine production, but not the test substance, was contacted with the cells. Although any inflammatory cytokine can be selected as the cytokine to be measured, IFN-γ, TNFα, IL-1, and IL-6 are preferred examples. By comparing the measured values thus obtained with the control and selecting wells in which the measured value of the well supernatant is lower than the control, a substance of interest can be selected from test substances. Since the substance thus selected possibly has a CD61-binding ability and has an inhibitory effect on the production of inflammatory cytokines, it can be used, as described above, as an inhibitor of inflammatory cytokine production or as a pharmaceutical for preventing or treating inflammatory diseases and hypercytokinemia.

After the above-described screening, a secondary screening is further performed and a substance whose activity depends on CD61 can be selected from the substances selected by the aforementioned screening method (hereinafter referred to as a primary selection substance). For example, CD61-expressing cells are seeded into 96-well plates and a primary selection substance and a labeled anti-CD61 antibody are added thereto. The supernatant of each well is used as a sample, a flow cytometry is performed on this sample, and a sample with a lower reaction than the control is selected. Herein, a control is a sample of CD61-expressing cells to which the labeled anti-CD61 antibody has been added, but not the primary selection substance. As a result of the flow cytometry, it can be conceived that, in a sample with a lower reaction than the control, the primary selection substance in the sample competitively inhibits the binding of the anti-CD61 antibody to CD61 on the cell surface. Accordingly, a substance selected by this secondary screening can be said to have a CD61-dependent inhibitory effect on the production of inflammatory cytokines.

All prior art documents cited herein are incorporated by reference.

### Examples

Herein below, the present invention is specifically described with reference to Examples, but it is not to be construed as being limited thereto.

### [Example 1] Preparation of monoclonal antibodies

The acquisition of functional antibodies that endogenously regulate cytokines was attempted. First, PBMC used as the immunogen were prepared from human blood. Blood from healthy normal subjects were collected into heparin tubes, diluted two fold with RPMI (Sigma R8340), and layered on Histopaque 1077 (Sigma). After centrifugation (800 x G, 30 min), the intermediate layer (leukocyte fraction) was taken out, and erythrocytes were removed by treating with a hemolytic buffer (150 mM NH₄Cl, 1 mM KHCO₃, 0.1 mM Na₂EDTA, pH 7.2-7.4) to obtain the PBMCs.

Balb/c mice were used as immunized animals. After an injection of 50 µl of Complete Adjuvant (Freund, Iatron) on the day before the initiation of immunization, mice were immunized by injecting the above-described PBMCs in batches of 2.0-5.0 x 10⁵ cells each into the foot pad every five days for four times. Mouse foot lymph nodes excised after the immunization were disrupted to obtain the lymphocytes. These lymphocytes were fused with the P3U1 myeloma cells using polyethylene glycol (PEG, Nakalai).

After the hybridomas were cultured in an RPMI (Sigma) selection medium supplemented with 15% FCS and Streptomycin/Penicillin (Gibco BRL) with a final concentration of 5 ng/ml and comprising 2% HAT solution (Gibco BRL), the immunoglobulin-producing ability of the hybridomas was confirmed by a sandwich ELISA, and hybridomas producing immunoglobulins in their supernatant were selected. The sandwich ELISA was performed using an anti-mouse IgG antibody (BS-AU271) sold by MBL as the sensitizing antibody (sensitized at a final concentration of 2 µg/ml) and a peroxidase-labeled anti-mouse IgG antibody (IM-0817) as the detection antibody (diluted 500-fold for detection).

For the immunoglobulin-producing hybridomas, culture supernatants which react to PBMCs were selected by flow cytometry. Flow cytometry was performed by staining sample PBMCs (1.0 × 10⁵ cells/sample) with 50 µl of culture supernatant and using an FITC-labeled anti-mouse antibody (available from MBL) as the secondary antibody.

Hybridomas producing culture supernatants showing a reactivity toward PBMCs were subjected to the limiting dilution method to obtain monoclonal clones. After purification, the produced monoclonal clone heavy chain (about 55 kDa) and light chain (about 30 kDa) were confirmed by SDS-PAGE to be single bands. From clones confirmed to be monoclonal, a clone whose culture supernatant affects the cytokine production of PBMCs was selected, to obtain the clone producing antibody #33. The method for examining the effect on cytokine production is similar to the method described in Example 2.

The monoclonal antibody #33-producing clone was further cultured in RPMI comprising 10% FCS and with a final concentration of 5 ng/ml Streptomycin/Penicillin (Gibco BRL), and culture supernatants for purification were obtained. IgGs in the culture supernatant of the hybridomas (mouse lymphocyte/P3U1) were purified using a Protein A column (Amersham Pharmacia). 50 mM Tris-HCl (pH 8.8), 3M NaCl was used as the washing buffer and 0.17 M Glycine-HCl (pH 2.3) was used as the elution buffer. The eluted IgGs were dialyzed using 50% glycerol/PBS. Antibody #33 was filter-sterilized using a 0.22 µm membrane (Millipore).

The antibody isotype was determined using the Isostrip kit (Roche). The isotype of antibody #33 was IgG2a, κ.

### [Example 2] Analysis of antibody #33's effect on cytokine production by PBMCs

To examine antibody #33's effect on cytokine production by PBMCs, PBMCs were cultured with cytokine production-stimulating substances, antibody #33 was added thereto, and the level of the various cytokines produced by PBMCs was examined.

PBMCs isolated from peripheral blood using Histopaque-1077 (Sigma) as described above were used. The isolated cells were seeded into 96-well plates at 1.0 ×10⁵ cells/well. RPMI (Sigma) into which Streptomycin/Penicillin (Gibco BRL) and 10% FCS (Equitech) have been added was used as the medium.

As cytokine production-stimulating substances, the CpG motif portion of a non-methylated DNA (GAC30: ACC GAT GAC GTC GCC GGT GAC GGC ACC ACG; custom synthesis requested to QIAGEN), LPS (Sigma) which is a bacterial component of Gram-negative bacteria, and Poly(I:C) (Sigma) which is a double-stranded viral RNA analogue were used. Each stimulating substance was added to the above-described cells under the conditions of final concentrations of 10 µM CpG, 100 ng/ml LPS, and 100 ng/ml Poly(I:C) and cells were cultured for two nights at 37°C under 5% CO₂. Antibody #33 was added to the above-described cells at the same time as the cytokine production-stimulating substances. The culture supernatants were recovered, the production level of the various cytokines was measured using commercially available ELISA kits, and the effect of antibody #33 on cytokine production by PBMCs was investigated. IM-1743 (IFN-γ), IM-1121 (TNFα), IM-0755 (IL-1α), IM-1987 (IL-10), and IM-3582 (IL-1β) (all from MBL) were used as measurement kits.

Furthermore, the variation over time of the production level of cytokines by PBMCs was also investigated. Isolated PBMCs were cultured in 12-well plates at 1.0 ×10⁶ cells/well, a fixed amount of culture supernatant was sampled 1, 2, 5, 10, 22, 32, 44, 56, 68, and 80 hours after addition of the stimulant, and the production level of the various cytokines was measured. Antibody #33 was added to the PBMCs at a final concentration of 1 µg/ml at the time of addition of stimulant. Furthermore, as another anti-CD61 antibody, a commercially available anti-CD61 antibody (SZ21, BC), and as a control, a mouse IgG2a (MBL) isotype control were each added to a final concentration of 1 µg/ml.

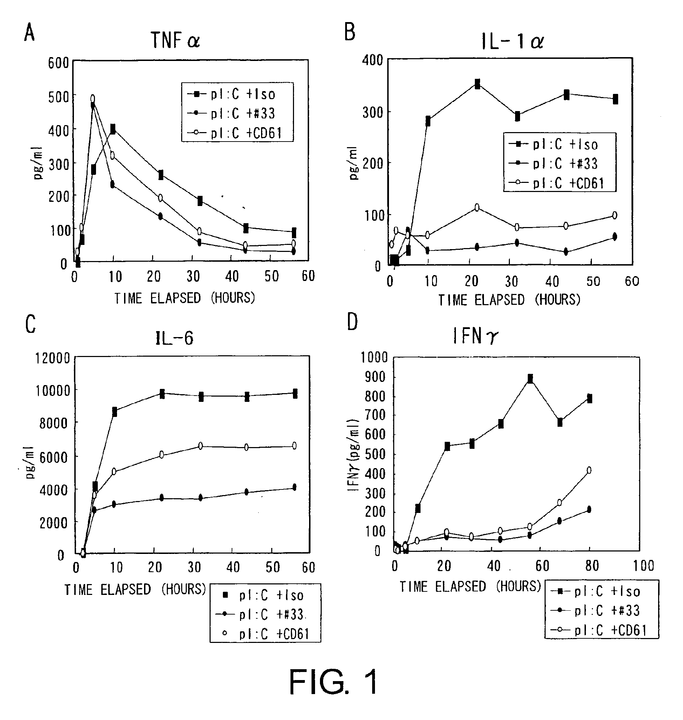

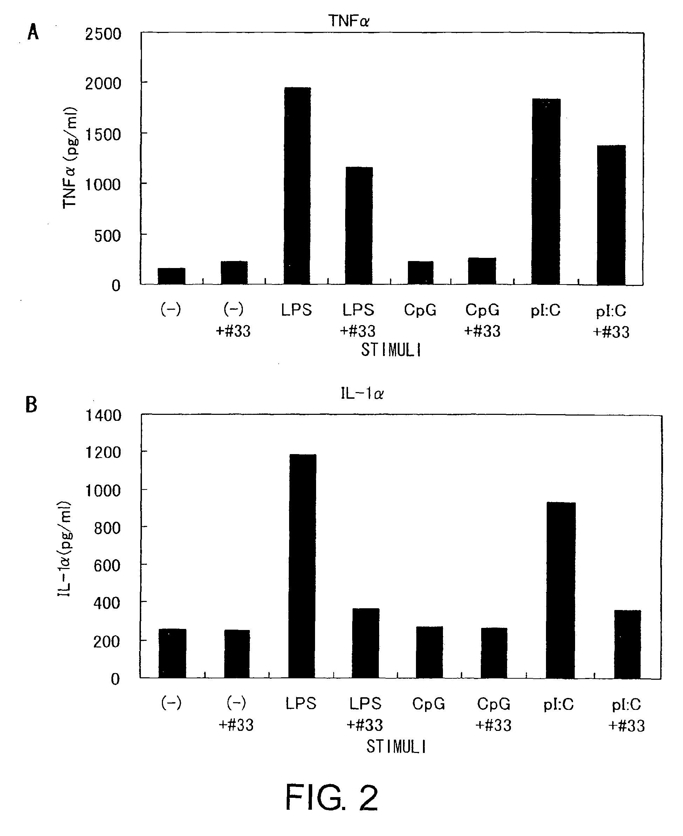

Results are shown in Figs. 1 to 3. Antibody #33, which is one of the monoclonal antibodies obtained this time, significantly inhibited IFN-γ production by PBMCs (Fig. 1D). Although IFN-γ production is usually detected on or after the 10^{th} hour after stimulation, IFN-γ production was barely observed in the presence of antibody #33 even after 40 hours had elapsed after the stimulation (Fig. 1D). Moreover, antibody #33 was found to also inhibit the production of TNFα and IL-1α, which are inflammatory cytokines (Fig. 2). Given this, a time course was also observed for the inhibitory effect on the production of TNFα and IL-1α, and furthermore on the inhibitory effect on the production of IL-6, which is a typical inflammatory cytokine. As for TNFα, addition of the antibody #33 or the anti-CD61 antibody accelerated the initiation time point of cytokine production; however, a value lower than the control was shown for the total production amount. As for IL-1α and IL-6, the production amount was inhibited from the time of addition of antibody #33 or the anti-CD61 antibody, similarly to IFN-γ (Fig. 1A to C). From these results, antibody #33 was found to be able to simultaneously inhibit the production of many types of inflammatory cytokines.

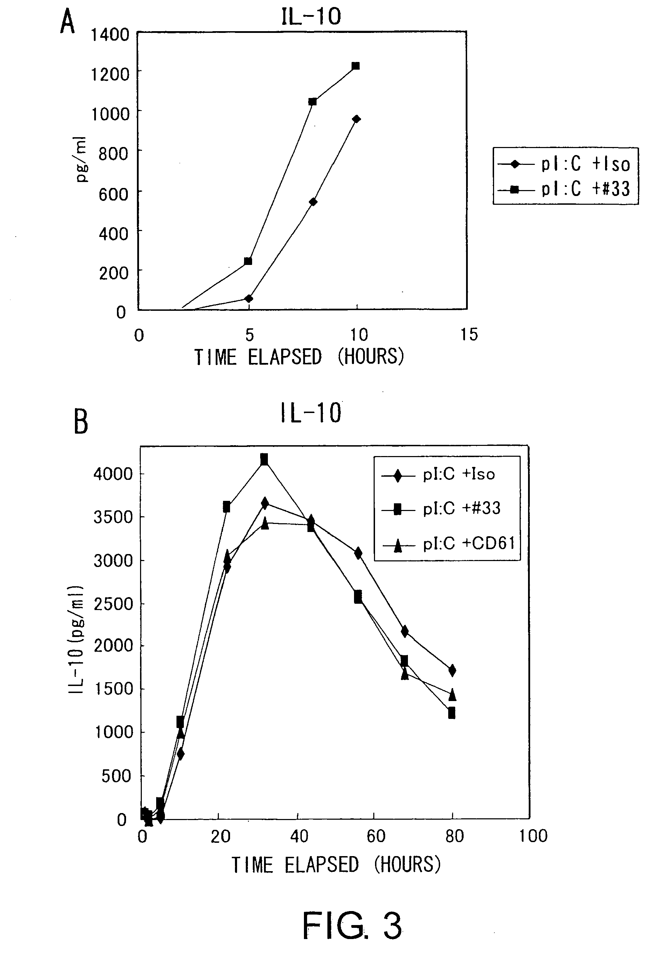

On the other hand, IL-10 is usually detected almost simultaneously with IFN-γ production or slightly later (by the 8^{th} to 10^{th} hour after stimulation). However, it was revealed that, when antibody #33 is administered simultaneously with the stimulation, the time point of initiation of production is by about five hours (Fig. 3A). However, as to the final IL-10 amount produced, no significant difference was observed compared to the control (Fig. 3B).

### [Example 3] Investigation of the involvement of IL-10 in the inhibitory effect on IFN-γ production by antibody #33

It is generally known that IL-10 inhibits the production of IFN-γ (Non-Patent Document 3). Given this, an investigation was carried out on whether IL-10 is involved in the promotion of IFN-γ production by antibody #33.

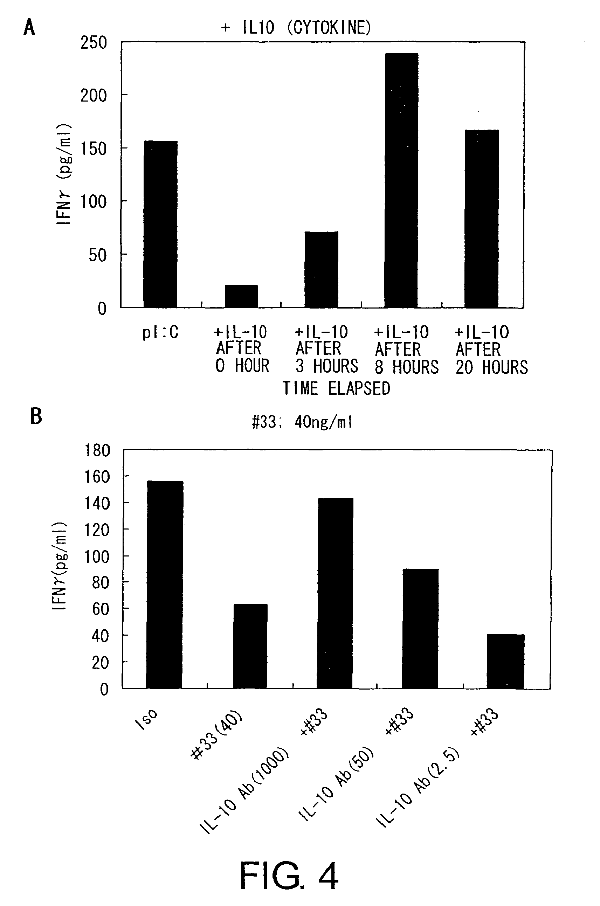

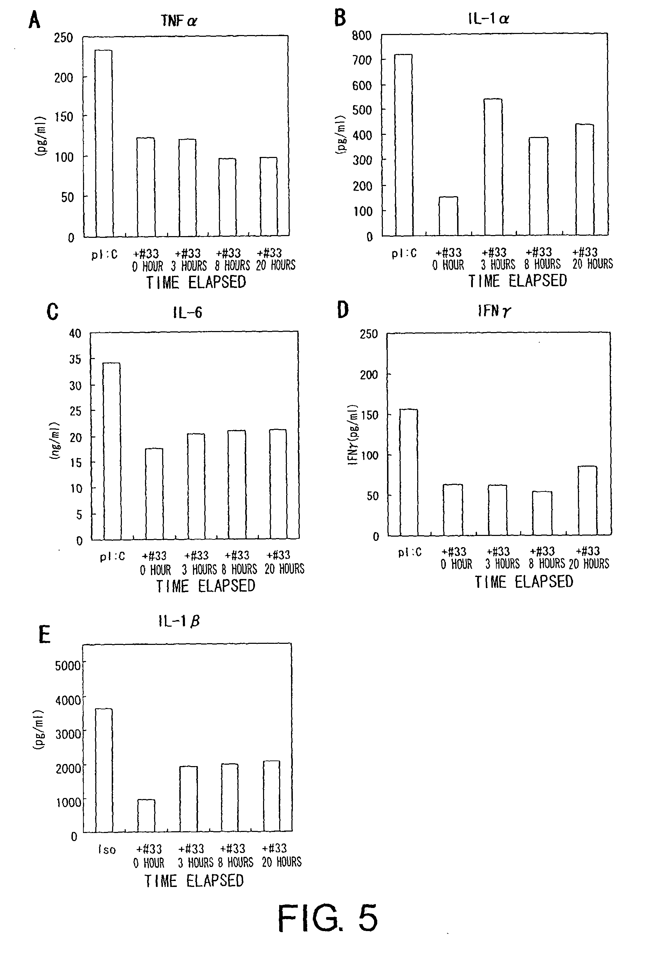

Poly(I:C) was added to PMBCs, then IL-10 was added at the time of PBMC addition (0 hr), three hours after addition (3 hrs), eight hours after addition (8 hrs), and 20 hours after addition (20 hrs), and the IFN-γ production level was measured at 48 hours after stimulation by ELISA. Antibody #33 was used instead of IL-10 and the production level of IFN-γ was similarly measured. IL-10 purchased from Sigma was used. Results are shown in Fig. 4A and Fig. 5. When IL-10 was added simultaneously to the stimulation of the PBMCs, IFN-γ production by PBMCs was inhibited, similarly to the Example 2 in which antibody #33 was added. However, when IL-10 was added on and after the 8^{th} hour of stimulation, no inhibitory effect on IFN-γ production was observed (Fig. 4A). On the other hand, an inhibition of IFN-γ production was confirmed even with an administration at 20 hours after stimulation of the PBMCs in the case of antibody #33 (Fig. 5D). Furthermore, when the production level of TNFα, IL-1α, IL-1β, and IL-6 was similarly verified, the cytokine production level was similarly inhibited for these cytokines by the addition of antibody #33 some time after induction of cytokine production. When considering use of antibody #33 in sepsis, this result suggests that antibody #33 would function more effectively than exogenous IL-10 administration in sepsis patients who have already developed hypercytokinemia.

Furthermore, Poly(I:C), antibody #33 and a IL-10-neutralizing antibody (R&D, MAB217) were simultaneously added to PBMCs and the produced IFN-γ was measured by ELISA 48 hours after stimulation. Antibody #33 was added at 40 ng/ml, while the IL-10-neutralizing antibody was added at 1000 ng/ml, 50 ng/ml, and 2.5 ng/ml. The result is shown in Fig. 4B. The inhibition of IFN-γ production by antibody #33 was suppressed in a concentration-dependent manner.

These results suggest that the inhibitory effect on IFN-γ production by antibody #33 depends on IL-10. In other words, it is thought that when antibody #33 is administered to stimulated PBMCs, the production of IL-10 is promoted, and as a result the production of IFN-γ and inflammatory cytokines is inhibited. Furthermore, antibody #33 is different from IL-10 in that the effect of its addition is recognized even when the period from PMBC stimulation is long; therefore, antibody #33 is assumed to suppress IFN-γ production by PBMC through some other pathway as well, in addition to its IL-10 production promoting effect.

### [Example 4] Identification of the antigen of antibody #33

To identify the antigen of antibody #33, its reactivity to various human cultured cells was examined by flow cytometry. THP-1 activated macrophage-like cells, HPB-ALL, Jurkat, PM-1, Molt-4, KM-3, HL-60, K562, THP-1, U937, 293T, and HUVEC were used as these cells. The THP-1 activated macrophage-like cells were prepared by culturing and differentiating for two days the THP-1 human monocyte-like cell line in RPMI (Sigma) including Streptomycin/Penicillin (Gibco BRL) and 10% FCS (Equitech) into which phorbol ester (PMA) (Sigma) has been added to a final concentration of 5 ng/ml .

Blocking with 1 mg/ml human IgG (Sigma) in FCS was performed for the various human cultured cells (1.0x10⁵ cells) and cells were reacted in a PBS solution comprising 2% FCS (Equitech) antibody #33 at a final concentration of 1 µg/ml at 4°C for 30 minutes. After washing twice with PBS comprising 2% FCS (Equitech), cells were further reacted with an FITC-labeled anti-mouse IgG secondary antibody (MBL) at 4°C for 30 minutes. Samples were further washed twice with PBS containing 2% FCS (Equitech) and analyzed with a flow cytometer (ALTRA BC).

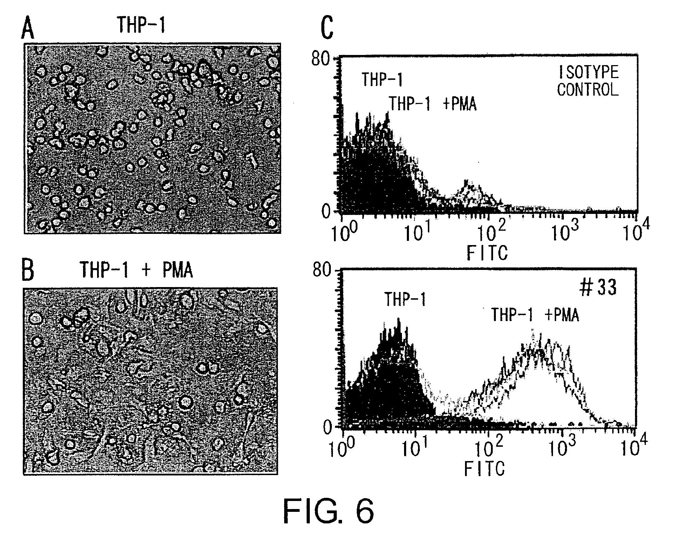

The results are shown in Fig. 6. It was revealed that antibody #33 strongly reacts with the THP-1 macrophage-like cells differentiated by phorbol ester (Fig. 6C). Furthermore, the T cell line Molt-4 and the human umbilical cord endothelial cells HUVEC also showed reactivity toward antibody #33.

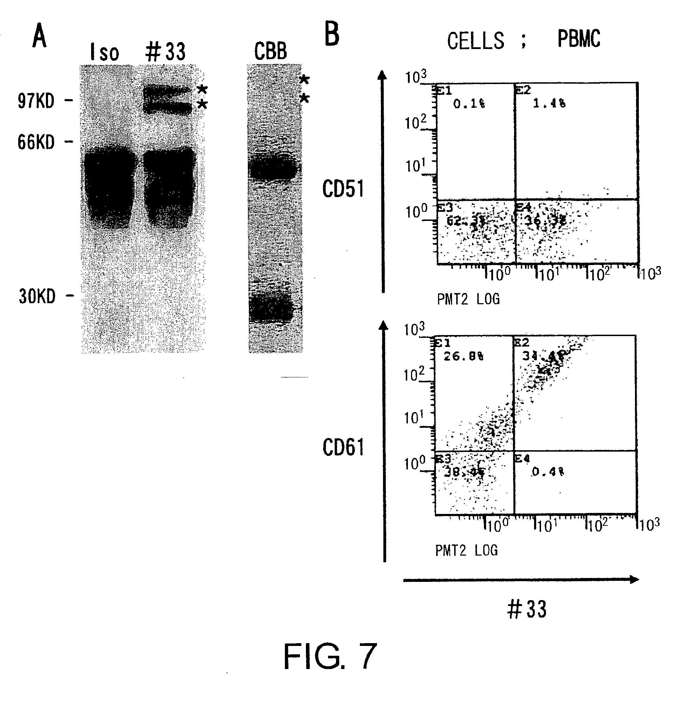

An immunoprecipitation experiment using antibody #33 was performed with these THP-1 cells to detect and isolate candidate antigen molecules. As a control, an isotype control was used. First, PBS containing 0.5 mM NHS-LC-Biotin (Pierce) was added to the THP-1 cells so that the cell density becomes 1.0 x 10⁷ cells/ml and this was reacted at 4°C for 1 hour to label the cell surface proteins. After the biotinylated cells were thoroughly washed cell surface proteins were solubilized with PBS containing NP-40 (Nacalai Tesque) and a protease inhibitor cocktail (Sigma). The solubilized proteins were mixed for one hour with Protein A (Amersham Pharmacia) onto which Human IgG (Sigma) have been adsorbed and non-specifically reacting proteins were precipitated and removed. Antibodies were added to the supernatants after the precipitation treatment, let to react at 4°C for one hour or more, and proteins bound to the antibodies were precipitated with Protein A sepharose (Amersham Pharmacia). Proteins adsorbed onto Protein A were eluted, an SDS-PAGE was carried out, and the labeled proteins of interest were detected using Avidine-POD (MBL) and ECL (Amersham Pharmacia). Detection by CBB staining was also performed. As a result, two specific bands were detected at around the molecular weight of 100 kDa. The results are shown in Fig. 7A.

From the above-described SDS-PAGE gel, the aforementioned two bands were excised and their analyses were outsourced to APRO Life Science Institute Inc. As a result of the enzymatic digestion (lysyl endopeptidase) of the gel fragments and the LC-MS/MS analysis and Mascot Search thereafter, it was revealed that, of the two bands, the upper band is CD51 (112 kDa) and the lower band is CD61 (84 kDa).

A flow cytometry was performed to determine which of CD51 and CD61, listed as candidate antigen molecules for antibody #33, is the antigen for antibody #33. After PBMCs were stained with antibody #33 and a secondary antibody, they were reacted with a PE-labeled CD51 antibody (BC) or a PE-labeled CD61 antibody (SZ21, BC) at 4°C for 30 minutes and double-stained, after which flow cytometry was performed.

The results are shown in Fig. 7B. No cell population double-stained with CD51 and antibody #33 was detected, while the staining properties of CD61 (SZ21, Beckman Coulter) and antibody #33 matched. From this result, it was concluded that the antigen for antibody #33 is CD61. Furthermore, as shown in Fig. 7B, antibody #33 and clone SZ21 do not mutually inhibit the staining, therefore, it was revealed that the epitope recognized by antibody #33 and the epitope recognized by the anti-CD61 antibody SZ21 are different.

On the other hand, although its epitope is different from that of antibody #33, an activity to remarkably inhibit the IFN-γ production by PBMCs was also observed with the anti-CD61 antibody SZ21 (Fig. 2A). From this result, it can be inferred that, to inhibit IFN-γ production with an anti-CD61 antibody, it is important to initiate a signal to CD61-expressing cells via CD61 (Integrin βIII), regardless of the epitopes.

### [Example 5] Examination of CD61-expressing cells

CD61-expressing cells were examined. Among PBMCs, CD61 is known to be expressed on monocytes (Non-Patent Document 7). Antibody #33-positive cells and antibody #33-negative cells among the PBMCs were separated and collected using a cell sorter, and the ability to produce the various cytokines was examined. As a result, it was revealed that antibody #33-positive cells produce IL-10 but not IFN-γ. Conversely, it was revealed that antibody #33-negative cells do not produce IL-10 but produce IFN-γ (data not shown). Considering that IFN-γ is mainly produced by Th1 in peripheral blood, a mechanism can be conceived, in which antibody #33 acts on monocytes to promote IL-10 production, the differentiation of naive T-cells (Th0) into Th1 is thereby inhibited, and consequently, the IFN-γ production level is decreased.

### [Example 6] Effect of antibody #33 on platelet aggregation

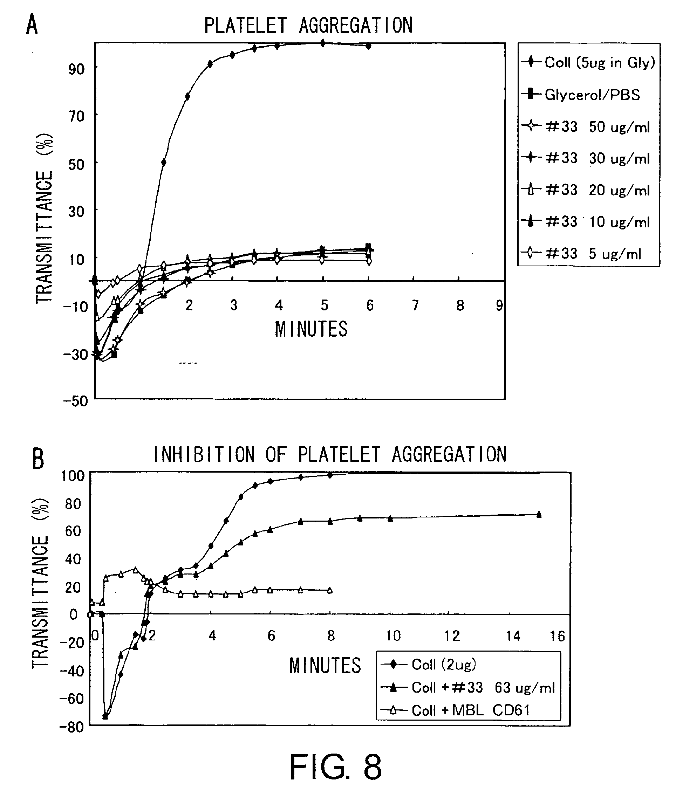

Since CD61 is strongly expressed on platelets, the effect of antibody #33 on platelet aggregation was examined. Blood (anticoagulant: sodium citrate) was centrifuged at room temperature at 800 rpm for 15 minutes to obtain PRP (Platelet Rich Plasma) as a supernatant. For the control, blood was centrifuged at room temperature at 2300 rpm for 15 minutes to obtain PPP (Platelet Poor Plasma) as a supernatant. Antibody #33 at final concentrations of 0, 5, 10, 20, 30, and 50 µg/ml or collagen (a coagulation-promoting substance) was added to the above-described PRP and the transmittance was monitored over time (for a few minutes) using a platelet aggregometer (NBS, HEMA TRACER). Since the transmittance increases when platelets aggregate, the degree of aggregation can be known from the obtained transmittance. As a result, no platelet aggregation activity was detected with antibody #33 (Fig. 8A).

To examine the effect of antibody #33 on collagen-induced platelet aggregation, the platelet-aggregating substance collagen (final concentration of 2 µg/ml) was added to PRP, antibody #33 was further added, and measurements using a platelet aggregometer were carried out. As a control, CD61 (MBL CD61) which has a platelet aggregation-inhibitory function was added instead of antibody #33 and measurements were similarly carried out. As for collagens inhibitory effect on aggregation, a slight aggregation inhibition was found (Fig. 8B). These results revealed that antibody #33 has no activity to cause platelet aggregation, but rather has an activity to inhibit aggregation.

### [Example 7] Examination of antibody #33's inhibitory effect on IFN-γ production ability in the presence of platelets

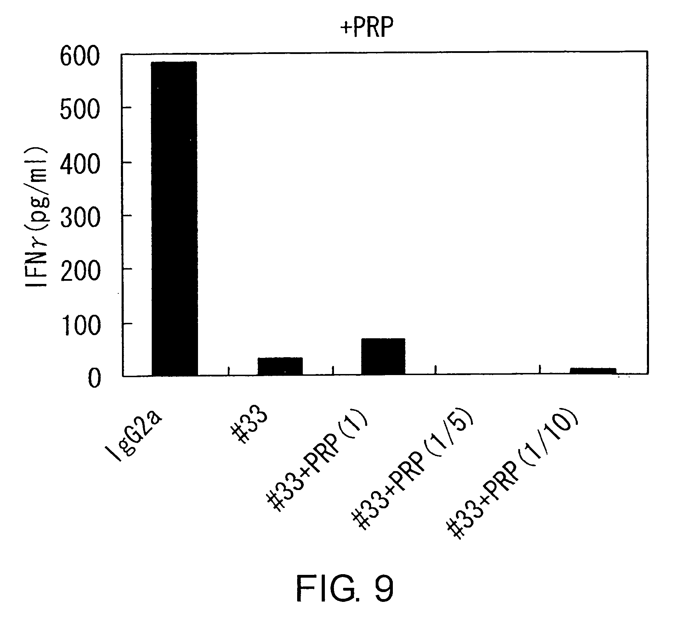

As described above, since CD61 strongly reacts with platelets, it was examined whether the inhibitory effect by antibody #33 on the IFN-γ production ability is shown even in the presence of platelets. A stimulating substance (Poly(I:C)), antibody #33, a culture medium containing platelets (PRP) were simultaneously added to PBMCs and the produced IFN-γ was measured. PRP was prepared in the same way as in Example 6 and used after replacing plasma with RPMI containing 10% FCS. Antibody #33 was added to the culture medium at a final concentration of 1 µg/ml. PRP was added to the culture medium such that PBMC: PRP (volume) becomes 1:1, 5:1, and 10:1. IFN-γ production level was measured at 48 hours after the addition and as a result, it was confirmed that antibody #33 inhibits IFN-γ production even in the presence of platelets (Fig. 9).

### [Example 8] Examination of the inhibitory effect on inflammatory cytokine production by the F(ab')₂ fragments of antibody #33

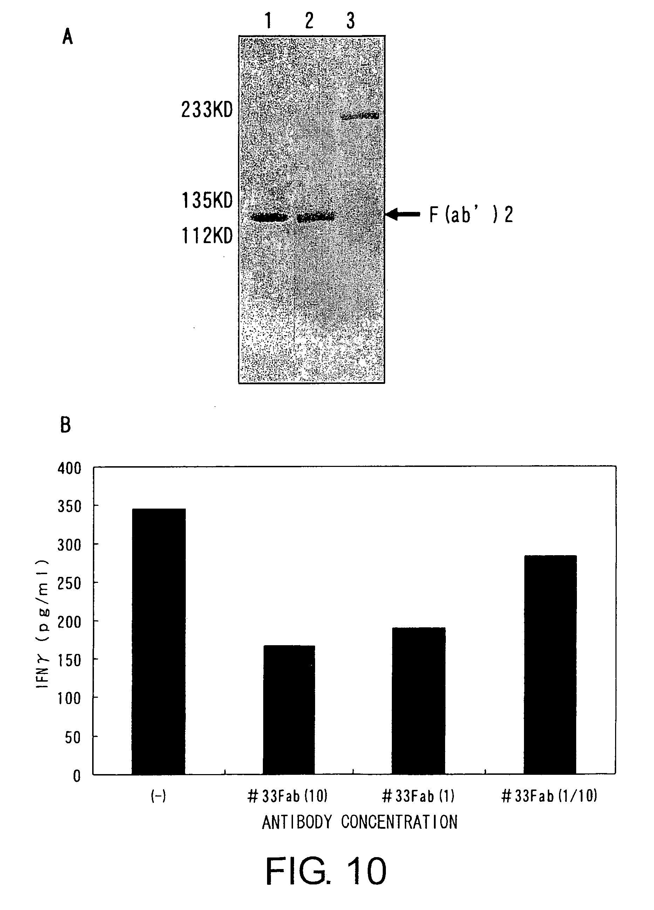

If antibody #33 is administered as is to the human body, it would bind to platelets, and the secondary effects arising from the antibody Fc portions becoming targets of macrophages cannot be ignored. Given this, F(ab')₂ fragments of the antibody were prepared and their inhibitory effect on IFN-γ production was examined (Fig. 10A).

Antibody #33 IgGs were purified as in the above-described Example 1, then treated with pepsin-agarose (Sigma) in 0.1 M acetic acid (pH 4.3) at 37°C for 20 hours to obtain F(ab')₂ fragments. After pepsin degradation, undigested materials having Fc portions were adsorbed with Protein A (Amersham Pharmacia) and the F(ab')₂ fraction was purified. After an SDS-PAGE was carried out under non-reducing conditions with the purified F(ab')₂ fragments of antibody #33 using a 7.5% acrylamide gel, a CBB staining was performed, and the molecular weight and the degree of purification were verified. When an SDS-PAGE is performed under non-reducing conditions, pepsin-undigested materials can be detected at around 200 kDa while F(ab')₂ fragments can be detected at around 150 kDa. Therefore, regarding the molecular weight and the degree of purification of purified F(ab')₂ fragments, it was verified whether purified products can be detected at 150 kDa and whether undigested materials (200 kDa) are mixed. The reactivity of the purified F(ab')₂ fragments of antibody #33 was verified by flow cytometry (data not shown).

Purified F(ab')₂ fragments of antibody #33 were added to PBMCs simultaneous to poly(I:C) addition, and the IFN-γ production level after 48 hours was measured by ELISA. F(ab')₂ fragments of antibody #33 were added at a final concentration of 10 µg/ml, 1 µg/ml, or 0.1 µg/ml. An inhibitory activity on IFN-γ production was confirmed also with the F(ab')₂ fragments of antibody #33, the results of which are indicated in Fig 10B. These results show that the inhibitory effect on IFN-γ production can indeed be expected even in the case of administration of F(ab')₂ fragments of antibody #33 to peripheral blood.

### [Example 9] Determination of the sequences of the variable regions and the CDRs of antibody #33

After collecting and disrupting 1x10⁷ cells from the total amount of antibody #33-producing hybridomas, a total amount of 6.0 µg of mRNAs was collected and purified using the Fast Track 2.0 kit (Invitrogen). A PCR was performed using the cDNAs synthesized from these mRNAs (Time Saver, Amersham) as templates and the heavy chain gene and light chain gene of the antibody were amplified. The primers used are shown below:
Forward primer for heavy chain amplification: ATGGRATGGAGCKGGRTCTTTMTCTT (SEQ ID NO: 17),
Reverse primer for heavy chain amplification: CAGTGGATAGACCGATGGGGC (SEQ ID NO: 18),
Forward primer for light chain amplification:
   ATGGATTTWCAGGTGCAGATTWTCAGCTTC (SEQ ID NO: 19),
Reverse primer for light chain amplification: ACTGGATGGTGGGAAGATGG (SEQ ID NO: 20).
Ambiguity code: R= A or G, K= G or T, M= A or C, W= A or T.
The amplified PCR products were purified (PCR purification kit, QIAGEN), then each one was cloned into pBluescriptIISK+ (STRATAGENE), and a sequence analysis was performed (SEQ2000, Beckman Coulter). Sequences obtained by the above-described analysis were compared with the known sequences (Sequences of Proteins of Immunological Interest FIFTH EDITION 1991, Elvin A. Kabat et al, NIH Publication No. 91-3242) to determine the CDRs.

### Industrial Applicability

The present invention has provided novel inflammatory cytokine inhibitors. Substances provided by the present invention can be used as useful pharmaceuticals in preventing or treating inflammatory diseases and hypercytokinemia via an inhibitory effect on inflammatory cytokines.

## Claims

1. A substance, or a derivative thereof, having an ability to bind to a CD61 protein and an inhibitory effect on inflammatory cytokine production.

2. The substance or derivative of claim 1, wherein the substance is a protein.

3. The substance or derivative of claim 1, wherein the substance is an antibody.

4. The substance or derivative of claim 1, 2, or 3, wherein the inflammatory cytokine is any one of IFN-γ, TNFα, IL-1, and IL-6.

5. The substance or derivative of claim 1, 2, 3, or 4, having an IL-10 production-inducing effect.

6. An inhibitor of inflammatory cytokine production comprising as an effective ingredient the substance or derivative of any one of claims 1 to 5.

7. An isolated DNA of any one of the following (a) to (d):
(a) a DNA of any one of SEQ ID NOs: 9 to 16;
(b) a DNA encoding the amino acid sequence of any one of SEQ ID NOs: 1 to 8;
(c) a DNA which hybridizes under stringent conditions with a DNA of any one of SEQ ID NOs: 9 to 16; and
(d) a DNA encoding an amino acid sequence with a deletion, addition, insertion and/or substitution of one or more amino acids in the amino acid sequence of SEQ ID NOs: 1 to 8.

8. A vector carrying the DNA of claim 7.

9. A transformant carrying the DNA of claim 7 or the vector of claim 8.

10. An anti-CD61 antibody, wherein the heavy chain polypeptide is a polypeptide of the following (a) or (b):
(a) a polypeptide comprising the amino acid sequence of SEQ ID NO: 4; or
(b) a polypeptide comprising an amino acid sequence with a deletion, addition, insertion and/or substitution of one or more amino acids in the amino acid sequence of SEQ ID NO: 4 and wherein the amino acid is functionally equivalent to the amino acid sequence of SEQ ID NO: 4.

11. An anti-CD61 antibody comprising the amino acid sequence of the following (a) or (b) as an amino acid sequence of a CDR (complementarity determining region) of a heavy chain polypeptide:
(a) an amino acid sequence of any one of SEQ ID NOs: 1 to 3; or
(b) an amino acid sequence with a deletion, addition, insertion and/or substitution of one or more amino acids in the amino acid sequence of SEQ ID NOs: 1 to 3 and which is functionally equivalent as a CDR (complementarity determining region) to the amino acid sequence of any one of SEQ ID NOs: 1 to 3.

12. An anti-CD61 antibody, wherein a light chain polypeptide is a polypeptide of the following (a) or (b):
(a) a polypeptide comprising the amino acid sequence of SEQ ID NO: 8; or
(b) a polypeptide comprising an amino acid sequence with a deletion, addition, insertion and/or substitution of one or more amino acids in the amino acid sequence of SEQ ID NO: 8 and wherein the amino acid is functionally equivalent to the amino acid sequence of SEQ ID NO: 8.

13. An anti-CD61 antibody comprising the amino acid sequence of the following (a) or (b) as an amino acid sequence of a CDR (complementarity determining region) of a light chain polypeptide:
(a) an amino acid sequence of any one of SEQ ID NOs: 5 to 7; or
(b) an amino acid sequence with a deletion, addition, insertion and/or substitution of one or more amino acids in the amino acid sequence of SEQ ID NOs: 5 to 7 and which is functionally equivalent as a CDR (complementarity determining region) to the amino acid sequence of any one of SEQ ID NOs: 5 to 7.

14. A pharmaceutical for preventing or treating an inflammatory disease, wherein the pharmaceutical comprises the inhibitor of inflammatory cytokine production of claim 6 or the anti-CD61 antibody of any one of claims 10 to 13.

15. A pharmaceutical for preventing or treating hypercytokinemia, wherein the pharmaceutical comprises the inhibitor of inflammatory cytokine production of claim 6 or the anti-CD61 antibody of any one of claims 10 to 13.

16. A method for inhibiting inflammatory cytokine production using the substance or derivative of any one of claims 1 to 5 or the anti-CD61 antibody of any one of claims 10 to 13.

17. A method for judging the effectiveness of the pharmaceutical of claim 14 or 15 in treating an inflammatory disease or hypercytokinemia, wherein the method comprises the step of contacting a test sample with an anti-CD61 antibody.

18. A kit for judging the effectiveness of the pharmaceutical of claim 14 or 15 in treating an inflammatory disease or hypercytokinemia.

19. A method of screening for a substance having an ability to bind to a CD61 protein and an inhibitory effect on inflammatory cytokine production, wherein the method comprises the steps of:
(a) contacting an inducer of cytokine production and a test substance with a CD61-expressing cell; and
(b) measuring the inflammatory cytokine level, comparing it with that of a control contacted with only the inducer of cytokine production, and selecting a test substance that reduced the cytokine level produced.


SEQUENCE LISTING

<110> MEDICAL AND BIOLOGICAL LABORATORIES CO., LTD.

<120> INFLAMMATORY CYTOKINE INHIBITOR

<130> M3-A0305P

<150> JP 2004-010971
<151> 2004-01-19

<160> 20

<170> PatentIn version 3.3

<210> 1
<211> 5
<212> PRT
<213> Mus musculus

<400> 1

Gly Tyr Thr Met Asn
1               5

<210> 2
<211> 17
<212> PRT
<213> Mus musculus

<400> 2

Leu Ile Asn Pro Tyr Ser Gly Val Thr Thr Tyr Asn Gln Arg Phe Lys
1               5                   10                  15

Gly

<210> 3
<211> 12
<212> PRT
<213> Mus musculus

<400> 3

Arg Gly Ala Leu Gly Gln Ala Tyr Tyr Phe Asp Tyr
1               5                   10

<210> 4
<211> 151
<212> PRT
<213> Mus musculus

<400> 4

Met Glu Trp Ser Gly Val Phe Ile Phe Leu Leu Ser Gly Thr Thr Gly
1               5                   10                  15

Val His Ser Glu Val Gln Leu Leu Gln Ser Gly Pro Glu Leu Val Lys
            20                  25                  30

Pro Gly Ala Ser Met Lys Ile Ser Cys Lys Ala Ser Gly Tyr Ser Phe
        35                  40                  45

Thr Gly Tyr Thr Met Asn Trp Val Lys Gln Ser His Gly Lys Asn Leu
    50                  55                  60

Glu Trp Ile Gly Leu Ile Asn Pro Tyr Ser Gly Val Thr Thr Tyr Asn
65                  70                  75                  80

Gln Arg Phe Lys Gly Lys Ala Thr Leu Thr Val Asp Lys Ser Ser Ser
                85                  90                  95

Thr Ala Tyr Met Glu Leu Leu Ser Leu Thr Ser Glu Asp Ser Ala Val
            100                 105                 110

Tyr Tyr Cys Ala Thr Arg Gly Ala Leu Gly Gln Ala Tyr Tyr Phe Asp
        115                 120                 125

Tyr Trp Gly Gln Gly Thr Thr Leu Thr Val Ser Ser Ala Lys Thr Thr
    130                 135                 140

Ala Pro Ser Val Tyr Pro Leu
145                 150

<210>  5
<211>  10
<212>  PRT
<213>  Mus musculus

<400>  5

Ser Ala Ser Ser Ser Val Asn Tyr Met His
1               5                   10

<210>  6

<211>  7
<212>  PRT
<213>  Mus musculus

<400>  6

Asp Thr Ser Lys Leu Ala Ser
1               5

<210>  7
<211>  9
<212>  PRT
<213>  Mus musculus

<400>  7

Gln Gln Trp Ser Ser Lys Pro Pro Thr
1               5

<210>  8
<211>  143
<212>  PRT
<213>  Mus musculus

<400>  8

Met Asp Leu Gln Val Gln Ile Ile Ser Phe Leu Leu Ile Ser Ala Ser
1               5                   10                  15

Val Met Ile Ser Arg Gly Gln Ile Val Leu Thr Gln Ser Pro Val Ile
            20                  25                  30

Met Ser Ala Ser Pro Gly Glu Arg Val Thr Leu Thr Cys Ser Ala Ser

35                  40                  45

Ser Ser Val Asn Tyr Met His Trp Tyr Gln Gln Lys Ser Gly Thr Ser
    50                  55                  60

Pro Lys Arg Trp Ile Tyr Asp Thr Ser Lys Leu Ala Ser Gly Val Pro
65                  70                  75                  80

Pro Arg Phe Ser Gly Ser Gly Phe Gly Thr Ser Tyr Ser Leu Thr Ile
                85                  90                  95

Thr Asn Met Glu Ala Glu Asp Ala Ala Thr Tyr Tyr Cys Gln Gln Trp
            100                 105                 110

Ser Ser Lys Pro Pro Thr Phe Gly Phe Gly Thr Lys Leu Glu Leu Gln
        115                 120                 125

Arg Ala Asp Ala Ala Pro Thr Val Ser Ile Phe Pro Pro Ser Ser
    130                 135                 140

<210>  9
<211>  15
<212>  DNA
<213>  Mus musculus

<400>  9
ggctacacca tgaac                                                    15

<210>  10

<211> 51
<212> DNA
<213> Mus musculus

<400> 10
cttattaatc cttacagtgg tgttactacc tacaaccaga ggttcaaggg c 51

<210> 11
<211> 36
<212> DNA
<213> Mus musculus

<400> 11
cggggggctc tgggacaggc gtactacttt gactac 36

<210> 12
<211> 453
<212> DNA
<213> Mus musculus

<400> 12
atggaatgga gcggggtctt tatcttcctc ctgtcaggaa ctacaggtgt ccactctgag 60

gtccagctgc tacagtctgg acctgagctg gtgaagcctg gagcttcaat gaagatatcc 120

tgcaaggctt ctggttactc attcactggc tacaccatga actgggtgaa gcagagccat 180

ggaaagaacc ttgagtggat tggacttatt aatccttaca gtggtgttac tacctacaac 240

cagaggttca agggcaaggc cacattaact gtagacaagt catccagcac agcctacatg 300

gagctcctca gtctgacatc tgaggactct gcagtctatt actgtgcaac acggggggct 360

ctgggacagg cgtactactt tgactactgg ggccaaggca ccactctcac agtctcctca 420

gccaaaacaa cagccccatc ggtctatcca ctg 453

<210> 13
<211> 30
<212> DNA
<213> Mus musculus

<400> 13
agtgccagct caagtgtaaa ttacatgcac 30

<210> 14
<211> 21
<212> DNA
<213> Mus musculus

<400> 14
gacacatcca aactggcttc t 21

<210> 15
<211> 27
<212> DNA
<213> Mus musculus

<400> 15
cagcagtgga gtagtaagcc acccacg 27

<210> 16
<211> 429
<212> DNA
<213> Mus musculus

<400> 16
atggatttac aggtgcagat tatcagcttc ctgctaatca gtgcctcagt catgatttcc 60

agaggacaaa ttgttctcac ccagtctcca gttatcatgt ctgcatctcc tggggagagg 120

gtcaccttga cctgcagtgc cagctcaagt gtaaattaca tgcactggta ccagcagaag 180

tcaggcacct cccccaaaag atggatttat gacacatcca aactggcttc tggagtccct 240

cctcgcttca gtggcagtgg gttcgggaca tcatactctc tcacaatcac caacatggag 300

gctgaagatg ctgccactta ttactgccag cagtggagta gtaagccacc cacgttcggt 360

tttgggacca agctggagct gcaacgggct gatgctgcac caactgtatc catcttccca 420

ccatccagt 429

<210> 17
<211> 26
<212> DNA
<213> Artificial

<220>
<223> An artificially synthesized primer sequence

<400> 17
atggratgga gckggrtctt tmtctt 26

<210> 18
<211> 21
<212> DNA
<213> Artificial

<220>
<223> An artificially synthesized primer sequence

<400> 18

cagtggatag accgatgggg c 21

<210> 19
<211> 30
<212> DNA
<213> Artificial

<220>
<223> An artificially synthesized primer sequence

<400> 19
atggatttwc aggtgcagat twtcagcttc 30

<210> 20
<211> 20
<212> DNA
<213> Artificial

<220>
<223> An artificially synthesized primer sequence

<400> 20
actggatggt gggaagatgg 20